(19) Europäisches Patentamt
European Patent Office
Office européen des brevets

(11) EP 0 768 997 B1

(12) **FASCICULE DE BREVET EUROPEEN**

(45) Date de publication et mention de la délivrance du brevet: **13.01.1999 Bulletin 1999/02**

(21) Numéro de dépôt: **95924997.0**

(22) Date de dépôt: **30.06.1995**

(51) Int. Cl.$^6$: **C07C 43/178**, C11D 1/72, C11B 9/00, C07C 43/11, C07C 43/196

(86) Numéro de dépôt international: **PCT/FR95/00883**

(87) Numéro de publication internationale: **WO 96/01245 (18.01.1996 Gazette 1996/04)**

(54) **DERIVES TERPENIQUES POLYALCOXYLES ET COMPOSITIONS EN CONTENANT**

POLYALKOXYLIERTE TERPENDERIVATE UND SIE ENTHALTENDE ZUSAMMENSETZUNGEN

POLYALKOXYL TERPENE DERIVATIVES AND COMPOSITIONS CONTAINING SAME

(84) Etats contractants désignés:
**AT BE CH DE DK ES FR GB GR IE IT LI LU NL PT SE**

(30) Priorité: **01.07.1994 FR 9408366**

(43) Date de publication de la demande: **23.04.1997 Bulletin 1997/17**

(73) Titulaire: **RHODIA CHIMIE
92408 Courbevoie Cédex (FR)**

(72) Inventeurs:
- **RICCA, Jean-Marc
F-69007 Lyon (FR)**
- **DERIAN, Paul-Noel
F-92260 Fontenay-aux-Roses (FR)**
- **HECAEN, Jean-Pierre
F-93240 Stains (FR)**
- **MERCIER, Jean-Michel
F-94320 Thiais (FR)**

(74) Mandataire:
**Fleurance, Raphael et al
Cabinet Beau de Loménie,
51, Avenue Jean Jaurès,
B.P. 7073
69301 Lyon Cédex 07 (FR)**

(56) Documents cités:
**DE-A- 3 822 202**
**FR-A- 2 429 213**
**US-A- 3 354 225**
**US-A- 3 370 080**
**US-A- 4 620 028**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 768 997 B1

Printed by Xerox (UK) Business Services
2.16.7/3.6

## Description

La présente invention concerne de nouveaux dérivés d'origine terpénique constitués par des cycloalcényles ou allq'les ayant au moins sept atomes de carbone et présentant des propriétés tensioactives et/ou parfumantes.

Outre cet aspect produit chimique nouveau, le domaine d'application de la présente invention est celui des composés tensioactifs et/ou parfumants.

## ART ANTERIEUR

Les composés, selon l'invention, appartiennent à une large famille chimique qui regroupe notamment et en premier lieu les dérivés du NOPOL ou 6,6-diméthylbicyclo[3.1.1]hept-2-ène-2-éthanol. Le NOPOL est un terpène obtenu en faisant réagir du pinène avec du formaldéhyde. Il est utilisé sous forme d'acétate de NOPOL dans le domaine de la pafumerie. Mais il n'a jamais été fait état d'une quelconque application de ce genre de composés dans le secteur des tensioactifs.

Le brevet japonais N° 61-233 650 divulgue des esters saturés du NOPOL et leurs dérivés. Ces esters répondent à la formule suivante :

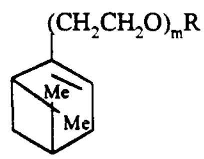

dans laquelle R est un reste acrylate et m = 1 ou 2.

Ces esters acryliques sont destinés à être utilisés comme monomeres dans la fabrication de poly(méth)acrylates, destinés à être employés comme matières premières dans la fabrication de lentilles optiques. Il s'agit donc ici de dérivés du NOPOL tout à fait particuliers. Ces dérivés appartiennent, certes, à la famille des cycloalcényles mais constituent un sous-ensemble spécifiquement limité au domaine restreint des poly(méth)acrylates.

On connaît également par la demande de brevet allemand N° 38 22 202 des esters acrylates de NOPOL de formule suivante :

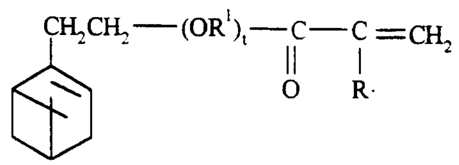

avec $R^1$ = alkyle en $C_2$-$C_4$, R = H ou $CH_3$ et t compris entre 0 et 10. Ces esters acrylates de NOPOL constituent des unités récurrentes d'homo ou de copolymères acrylates utiles comme additifs (durcisseurs) pour béton. Il s'agit là encore d'une structure chimique du type ester d'acrylate de NOPOL polyalkylènalcoxylé. Ces produits font eux aussi partie du sous-ensemble restreint et bien délimité des poly(méth)acrylates.

De la même façon que l'application fabrication de lentilles évoquée cidessus, l'application durcisseur pour béton n'a rien à voir avec les fonctionnalités tensioactives et/ou parfumantes, auxquelles on s'intéresse dans le cadre de l'invention.

Dans un domaine également aux antipodes de celui des tensioactifs, la demande de brevet français N° 2 429 213 divulgue, incidemment, un précurseur de dérivés de norpinane, qui sont utiles comme antispasmodiques et qui sont ceux visés par la demande. Ce précurseur est un produit isolé de formule 2-[2-(6,6-diméthylnorpinane-2-yl)éthoxy]éthanol. Cette demande de brevet ne fait aucune allusion aux propriétés spécifiques de cet intermédiaire de synthèse.

La famille chimique dont relève l'invention comprend également les dérivés de l'arbanol, composé terpénique dérivant du camphane et répondant à la formule suivante :

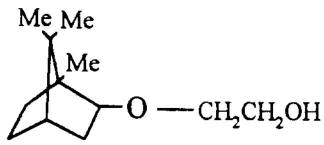

Les brevets américains N° 3 354 225 et 4 620 028 décrivent des dérivés du camphane éthoxy-hydroxylés (arbanol) ou propoxy-hydroxylés. Le greffon oxyallcylène selon l'US-A-4 620 028 peut comprendre 2 à 4 unités éthoxy ou propoxy.

Ces brevets US ne mentionnent pas de propriétés particulières pour ces dérivés du camphane, mis à part leur propriété parfumante.

L'art antérieur de l'invention se rapporte également au domaine de la détergence. Ainsi, parmi les tensioactifs connus, les plus largement mis en oeuvre à l'echelle industrielle, par exemple en détergence, sont notamment les alcools gras linéaires polyalcoxylés, tels que par exemple le dodécanol ou alcool laurique polyéthoxylé. A défaut de mieux, ces tensioactifs ne s'imposent qu'en raison du faible coût de revient de leur synthèse. En effet, les atouts de ces produits ne sont pas dans leur pouvoir tensioactif car celui-ci est relativement faible et ne donne donc pas entièrement satisfaction.

Contrairement à cela, les alkylphénol(poly)alcoxylés qui sont d'autres tensioactifs industriels, possèdent des propriétés tensioactives relativement bonnes mais demeurent d'un emploi limité, en raison de leur toxicité et de leurs effets nocifs sur l'environnement.

Par ailleurs, ces tensioactifs connus ont tous en commun un inconvénient majeur, à savoir : leur propension à produire de la mousse. Cela est particulièrement génant aussi bien dans la détergence industrielle que domestique.

Comme autre exemple d'art antérieur non pertinent, on peut citer le brevet américain US N° 3 370 080, qui divulgue des composés (revendication 5) du type bicycloheptyle substitués par des restes ω-hydroxypolyoxyalkylène. Ces restes comportent des motifs récurrents linéaires non ramifiés qui sont, soit uniquement en $C_2$, soit uniquement en $C_3$. Les bicycles, entrant dans la structure de ces composés, ne comprennent pas de carbone substitué par deux alkyles en $C_1$-$C_6$. Par ailleurs, il est à noter que ces composés connus ne sont pas présentés comme des détergents/parfumants, mais simplement comme des détergents aux performances modestes.

Dans cet état de connaissances, l'un des objectifs essentiels de la présente invention est de fournir de nouveaux composés chimiques doués notamment d'un bon comportement en tant que tensioactifs.

Un autre objectif de l'invention est de fournir de nouveaux composés chimiques tensioactifs qui soient d'obtention aisée et économique.

Un autre objectif de l'invention est de fournir de nouveaux composés tensioactifs d'une toxicité aussi faible que possible et bénéficiant, dans ce même registre, de la caractéristique avantageuse qui est de respecter l'environnement.

Un autre objectif de l'invention est de fournir de nouveaux composés chimiques tensioactifs ayant une odeur agréable et qui soient non moussants.

Un autre objectif de l'invention est de fournir un procédé, simple et peu onéreux, de préparation de nouveaux composés chimiques, notamment tensioactifs, du type de ceux évoqués ci-dessus.

## BREF EXPOSE DE L'INVENTION

C'est dans ce contexte que la Demanderesse a mené de longues et laborieuses recherches et expériences, au terme desquelles elle a eu le mérite de parvenir, de façon toute à fait surprenante et inattendue, à isoler une nouvelle classe de produits chimiques qui sont doués, notamment, de très bonnes qualités tensioactives, et qui appartiennent à la famille chimique des cycloalcényl (ou alcoyl-)-(éventuellement oxy)alkylényl-polyalcoxylés.

## DESCRIPTION DETAILLEE

Ainsi, la présente invention atteint les objectifs mentionnés ci-dessus, parmi d'autres, en proposant des composés de formule suivante :

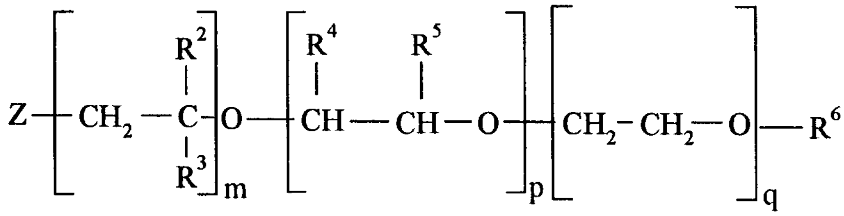

(I)

dans laquelle :

* Z représente un radical bicyclo[a,b,c]hepténylе ou bicyclo[a,b,c]heptyle, avec :

⊕ a + b + c = 5,
⊕ a = 2, 3 ou 4,
⊕ b = 2 ou 1,
⊕ c = 0, 1,

ledit radical étant éventuellement substitué par au moins un alkyl en $C_1$-$C_6$, de préférence un méthyle et comprenant un squelette choisi parmi ceux mentionnés ci-après (**Z**$_1$ à **Z**$_7$), ainsi que parmi leurs correspondants heptyle exempts de double liaison :

1) 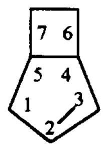 [3.2.0]

2) 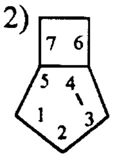 [3.2.0]

3) 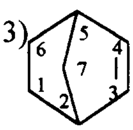 [2.2.1]

4) 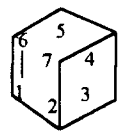 [3.1.1]

5) 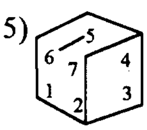 [3.1.1]

6) 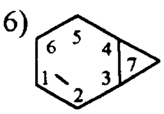 [4.1.0]

7) 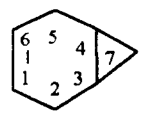 [4.1.0]

▷ $R^2$ et $R^3$ sont identiques ou différents et représentent l'hydrogène ou un groupement (cyclo)alkyle ou (cyclo)alcényle, linéaire ou ramifié, de préférence (cyclo)alkyle linéaire ou ramifié en $C_1$-$C_{22}$,
l'hydrogène et le méthyle étant plus particulièrement préférés;
▷ $R^4$ et $R^5$ sont identiques ou différents et représentant l'hydrogène ou un radical (cyclo)alkyl ou (cyclo)alcényle linéaire ou ramifié, de préférence (cyclo) alkyle linéaire ou ramifié en $C_1$-$C_{22}$,
l'hydrogène, le méthyle et l'éthyle étant plus particulièrement préférés,
▷ $R^6$ correspond à l'hydrogène ou à l'un des groupements suivants : $SO_3M$, $OPO_3(M)_2$, $-(CH_2)_m$--COOM avec m compris entre 1 et 6, $-(CH_2)_z$--SO $_3M$ avec z = 1 à 6 et dans lesquels M = H, Na, K, Li ou $N(R^7)_4{}^+$ avec $R^7$ = H ou un (cyclo)alkyle en $C_1$-$C_{22}$, de préférence en $C_1$-$C_6$, éventuellement hydroxylé,

l'hydrogène le méthyle et l'hydroxyéthyle étant préférés.

▷

. m = 0,1
. $1 \leq p \leq 20$ de préférence $1 \leq p \leq 10$, et plus préférentiellement encore $1 \leq p \leq 3$.
. $1 \leq q \leq 200$, de préférence $1 \leq q \leq 50$ et, plus préférentiellement encore, $1 \leq q \leq 10$.

▷ avec la condition selon laquelle quand m = 0, au moins l'un des substituants $R^4$, $R^5$ est différent de l'hydrogène.

Les composés d'origine terpénique selon l'invention forment une classe chimique particulière, qui constitue une sélection originale au sein de la famille des cycloalcényl ou alkyl-(éventuellement oxy)alkylènyl-polyalcoxylés. Les composés de cette classe présentent, notamment, des propriétés tensioactives et non moussantes et/ou parfumantes tout à fait intéressantes.

Dans la formule (**I**), le radical Z est, de préférence, rattaché au reste de la chaîne par l'intermédiaire de l'un quelconque de ses carbones $C_1$ à $C_6$, les carbones $C_1$, $C_5$ et $C_6$ étant plus préférentiellement sélectionnés.

Dans le cas où l'on a affaire à des bicyclo-hept-ane ou(-ène) reliés à une chaîne polyalcoxylée par l'intermédiaire d'un pontoxygène (m = 0), il est prévu que soit $R^4$, soit $R^5$ ne corresponde pas à l'hydrogène.

On peut donc avoir par exemple $R^4$ = H et $R^5$ = alkyle $C_1$-$C_6$, de préférence méthyle.

Par ailleurs, il doit être considéré que la position des motifs récurrents affectés des paramètres p et q dans le greffon de Z (Formule **I**), n'est pas limitative. La séquence représentée dans la formule (**I**) n'est qu'un exemple parmi d'autres.

Conformément à une modalité préférée de l'invention, les composés (**I**) sont caractérisés en ce que leur radical Z est substitué sur au moins l'un de ses carbones par au moins deux groupements alkyles en $C_1$-$C_6$, de préférence par deux $CH_3$, avantageusement localisé sur le carbone 7 de Z

Une première sous-famille de composés (**Ia**), plus particulièrement mise en oeuvre, est celle comprenant les composés (**I**) pour lesquels m = 1 et dont le radical Z est sélectionné dans la liste comportant les radicaux $Z_3$ à $Z_7$ définis ci-dessus et substitués sur le carbone 7 par deux méthyles.

Les squelettes $Z_4$ et $Z_5$ substitués en $C_7$ par deux $CH_3$ sont des squelettes Z privilégiés, dans la sous-famille (**Ia**).

Les composés (**Ia**) encore plus préférés selon l'invention sont des dérivés de terpène pour lesquels m = 1 dans la formule (**I**), particulièrement des dérivés de pinène et, plus particulièrement encore des dérivés de NOPOL.

Une deuxième sous-famille (**Ib**) intéressante de composés selon l'invention est celle comprenant les composés (**I**) pour lesquels m = 0 et dont le radical Z comprend le correspondant heptyle sans double liaison du squelette $\mathbf{Z_3}$.

Dans cette sous-famille (**Ib**), le correspondant heptyle de $\mathbf{Z_3}$ comprend avantageusement deux méthyles sur le carbone 7, ce correspondant étant, de préférence, substitué par au moins un reste alkyle - avantageusement en $C_1$-$C_6$ - de préférence par au moins un méthyle, sur le carbone **2** ou **5**. Dans le cas d'une substitution méthyle du carbone **5**, on est en présence d'un reste bornyle ou isobornyle selon la localisation dans l'espace du $CH_3$ en $C_5$.

Or, précisément les composés (**Ib**) préférés sont ceux pour lesquels m = 0 et Z = $Z_3$ du type (iso)bornyle :

($Z_3$ saturé) 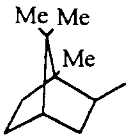

Avantageusement, les motifs récurrents de la formule (**I**) identifiés par les paramètres p et q sont présents en quantité telle et sont agencés de telle sorte qu'ils forment une chaîne polymère statistique ou séquencée, de préférence séquencée dans l'ordre donné par la formule (**I**).

On s'intéresse ainsi, plus spécifiquement, mais non limitativement, dans le cadre de l'invention, à des produits résultant de l'éthoxylation et/ou la propoxylation du NOPOL (**Ia**) et de l'ARBANOL (**Ib**). Ces réactions conduisent à la formation d'une chaîne de polyoxydes d'éthylène et/ou de polyoxydes de propylène, chaîne qui constitue le pôle hydrophile de la molécule tensioactive.

Les propriétés tensioactives de ces composés sont tout à fait appréciables. Ces propriétés peuvent être évaluées, e. g à l'aide de techniques d'analyse connues, reposant sur la mesure de la température de trouble en fonction de la Balance d'Hydrophilie Lipophilie (HLB).

Il a pu ainsi être observé que les composés selon l'invention possèdent un comportement tensioactif meilleur ou sensiblement équivalent à celui des tensioactifs connus (mais toxiques), que sont les nonylphénoléthoxylés. En outre, les performances tensioactives des composés de l'invention restent acceptables par rapport à celles des alcools gras linéaires, tels que les alcools lauriques.

Mais les qualités particulièrement avantageuses des composés selon l'invention, sur lesquelles il faut insister, est leur effet non moussant et leur défaut d'odeur désagréable.

Par ailleurs, il convient de souligner que les composés **(I)**, dont certains sont d'origine naturelle, sont relativement peu toxiques et sans effets nocifs sur l'environnement.

Un autre intérêt de ces composés **(I)** est qu'ils peuvent être obtenus relativement aisément avec un coût de revient acceptable.

Ainsi, les composés **(Ia)** **[m=1]**, à double fonctionnalité tensioactive/parfumante peuvent être préparés par mise en oeuvre d'un procédé consistant essentiellement à faire réagir un réactif de formule **(I')** :

**(I')**

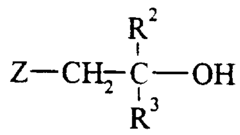

. avec un réactif **(Wop)** de formule :

**(Wop)**

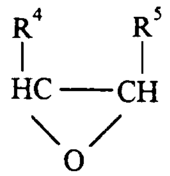

et, de préférence puis,

. avec un réactif **(Woe)** de formule.

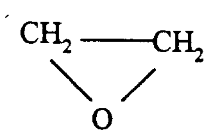

Formules dans lesquelles

- ▷ Z est tel que défini dans les revendications 1, 2, 3 ou 4,
- ▷ $R^2$ et $R^3$ sont identiques ou différents et représentent l'hydrogène, un (cyclo)alkyle, un (cyclo)alcényle linéaire ou ramifié en $C_1$-$C_{22}$, l'hydrogène et le méthyle étant plus particulièrement préférés,
- ▷ $R^4$ et $R^5$ sont identiques ou différents et représentent l'hydrogène ou un radical (cyclo)alkyl ou (cyclo)alcényle linéaire ou ramifié, de préférence (cyclo)alkyle linéaire ou ramifié en $C_1$-$C_{22}$, l'hydrogène, le méthyle et l'éthyle étant plus particulièrement préférés, avec la condition selon laquelle au moins l'un des substituants $R^4$, $R^5$ est différent de l'hydrogène.

pour obtenir un produit **(I.1)** :

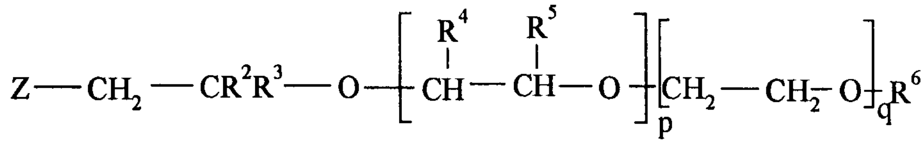

(I.1)

avec p, q et $R^6$ sont tels que définis supra.

Cette réaction s'effectue en présence d'une quantité efficace de catalyseur et à une température supérieure à 100° C, de préférence comprise entre 120° et 250° C et, plus préférentiellement encore, comprise entre 150° C et 200° C.

S'agissant des composés **(Ib)** pour lesquels m = 0, ils peuvent être préparés par le procédé consistant essentiellement à faire réagir un réactif de formule **(I")**:

(I'') 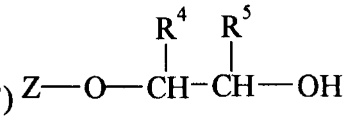

dans laquelle les définitions de $R^4$, $R^5$ correspondent à celles données supra; avec un réactif (**Wop**) de formule définie supra.
et, de préférence puis,
avec un réactif (**Woe**) de formule également définie supra ;
pour obtenir un produit (**I.2**) :

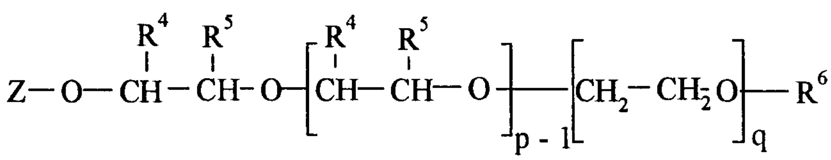

(I.2)

avec p, q et $R^6$ tels que définis supra.

Cette réaction s'effectue en présence d'une quantité efficace de catalyseur et à une température supérieure à 100° C, de préférence comprise entre 120° et 250° C et, plus préférentiellement encore, comprise entre 150° C et 200° C.

Les réactions (**I'**) ou (**I"**) + (**Wop** et **Woe**) sont éventuellement suivies d'une fonctionnalisation visant à transformer l'hydrogéne terminal en l'un des autres substituants $R^6$, tels que définis supra.

Les composés de formule générale (**I'**) peuvent être obtenus par réaction de condensation entre, d'une part, des précurseurs bicycliques allryléniques, portant une double liaison exocyclique et de structures voisines de celles des radicaux Z définis ci-dessus et, d'autre part, des composés portant une fonction carbonylée de type aldéhyde ou cétone.

Ces composés bicycliques sont. avantageusement, des bicyclo[a,b,c]n-alkylène(de préférence méthylène)heptanes avec n = 1 à 6 et a + b + c = 5 .

A titre d'exemples, de tels composés, on peut citer : le bicyclo[3.2.0]-2-méthylèneheptane, bicyclo[2.2.1]-2-méthylèneheptane, 2-méthylènenorbomane, 1-méthyl-2-méthylènenorbomane, isosantène, β pinène, ce dernier produit étant particulièrement préféré.

Parmi les composés à fonction carbonylée, on utilise, de préférence, les aldéhydes et, plus préférentiellement encore, les aldéhydes linéaires saturés, le formaldéhyde étant particulièrement préféré.

Le formaldéhyde peut être utilisé sous ses différentes formes. solution aqueuse, trioxanne ou paraformaldéhyde. Eventuellement, le formaldéhyde peut être employé sous forme gazeuse par dépolymérisation à chaud du paraformalédhyde.

La réaction de condensation peut s'effectuer en phase liquide ou gazeuse, sous pression atmosphérique ou autogène.

Conformément à une caractéristique originale de l'invention, le procédé d'obtention des produits (**I'**) consiste donc, essentiellement, en une condensation entre au moins un bicyclo[a.b.c]-n-alkylène heptane (avec n = 1 à 6 et a + b + c = 5 ) et au moins un composé carbonylé, ladite condensation s'effectuant avantageusement en l'absence de solvant et, éventuellement, en présence de catalyseur.

Il est également envisageable d'utiliser un solvant inerte. Parmi les solvants compatibles, on peut citer les solvants organochlorés comme le dichlorométhane, le chloroforme, le tétrachlorure de carbone, les solvants aromatiques comme le benzéne, le toluène, les xylènes, les chloroaromatiques, tels que chloro-et dichloro-benzénes, les solvants alcooliques, tels que les alcools linéaires ou branchés ($C_1$-$C_{22}$), les alcools cycliques comme le cyclohexanol, les solvants de type alcénique comme l'alpha pinéne ou le camphène.

En pratique, on peut ainsi prévoir que la réaction se déroule, par exemple, en présence d'alphapinène dans un rapport 0,5-10 moles d'alphapinéne/mole de précurseur.

Les produits (**I'**) sont généralement obtenus après distillation du solvant éventuel sous forme liquide ou solide et purifiés par distillation ou cristallisation.

Cette réaction peut être avantageusement améliorée par l'utilisation de catalyseurs homogènes ou hétérogènes, à base d'acides de Lewis homogènes ou supportés. Parmi les acides de Lewis, on peut citer les alkoxydes d'aluminium et de titane, les dérivés halogénés d'aluminium, titane, fer, zinc et bore.

En particulier, on préfère les dérivés du fer et du zinc comme le trichlorure de fer et le dichlorure de zinc. Plus préférentiellement encore; on utilise le dichlorure de zinc.

Ces catalyseurs sont employés dans un ratio de 0,001 à 0.5 mole de catalyseur par mole de précurseur, de préférence 0,01 à 0,1 mole/mole.

Les catalyseurs sont éliminés après réaction, par filtration ou lavage avec des solutions alcalines.

La réaction de condensation se conduit dans une plage de température de 50-300° C, en phase liquide sous pression autogène ou de 250-600° C en phase gazeuse. De préférence, elle est conduite en phase liquide de 120-240° C, de préférence de 150-200° C.

Le composé carbonylé est mis en oeuvre dans des rapports de 0,5-10 moles par mole de précurseur de (**I'**), de préférence 0,5-2.

L'excès éventuel peut être séparé après réaction par filtration ou distillation.

Il est à souligner que le composé (**I'**) correspondant aux composés présentant, respectivement, un radical $Z_4$ et $Z_5$, est le NOPOL.

Pour ce qui est des réactifs de (**Woe** et **Wop**), on fait appel, de préférence, à l'oxyde d'éthylène, à l'oxyde de propylène, à l'oxyde de butylène ou leurs mélanges. On préfère l'oxyde d'éthylène, l'oxyde de propylène ou leurs mélanges.

La quantité des réactifs à mettre en présence peut varier largement.

L'oxyde d'allcylène est généralement en excès par rapport à l'alcool aliphatique bicyclique (**I'**). Le rapport molaire, entre le nombre de moles d'oxyde d'alkylène et le nombre de moles d'alcool aliphatique bicyclique (**I'**), varie entre 1 et 50 et, plus préférentiellement, entre 1 et 10.

On effectue ensuite la réaction en présence d'une quantité efficace de catalyseur.

A titre de catalyseurs préférés, on peut mentionner les bases fortes et, plus particulièrement, les hydroxydes de métal alcalins ou alcalino-terreux, de préférence la soude ou la potasse ou les hydroxydes d'ammonium quaternaire, et notamment l'hydroxyde de tétraméthylammonium ; les alkoxydes alcalins ou alcalino-terreux, de préférence, le méthylate, l'éthylate ou le tertiobutylate de sodium ou de potassium.

On peut également utiliser, à titre de catalyseurs, les amines primaires, secondaires ou tertiaires, de préférence les amines aliphatiques : ces amines pouvant comporter d'autres fonctions, telles que des fonctions éther.

Comme exemple d'amines, on peut citer la N,N-diméthyllaurylamine.

Comme autres types de catalyseurs, on peut faire appel aux catalyseurs de type acide de Lewis. Comme exemples de catalyseurs convenant tout à fait bien, on peut citer : $BF_3$ sous forme gazeuse ou en solution dans le diéthyléther, le chlorure d'étain $SnCl_4$ ou le chlorure d'antimoine $SbCl_5$.

La quantité de catalyseur à mettre en oeuvre est déterminée selon la nature du catalyseur.

S'agissant d'un catalyseur basique, on exprime cette quantité par l'indice de base qui, est défini comme la quantité de base exprimée en mg, rapportée au poids de produit final.

Selon l'invention, l'indice de base est compris entre 0,5 et 40 mg, de préférence entre 0,5 et 5 mg.

Lorsque le catalyseur est acide, la quantité mise en oeuvre représente 0,1 à 10 mmoles par moles d'alcool aliphatique bicyclique (**I'**).

La température de la réaction est choisie de manière qu'elle soit suffisante pour permettre l'accomplissement de la réaction.

La température de la réaction est choisie, de préférence, supérieure à 100° C, de préférence comprise entre 120 et 250° C et, encore plus préférentiellement, entre 150 et 200° C.

Avantageusement, la réaction est conduite en présence d'un gaz inerte, qui peut être l'azote, ou d'un gaz rare, de préférence l'argon ou le monoxyde de carbone.

La réaction se déroule sous pression atmosphérique ou à une pression inférieure ou supérieure à celle-ci. Ainsi, la réaction peut être conduite sous pression réduite, par exemple comprise entre 200 et 700 mm de mercure ($2,7.10^4$ à $9,3.\ 10^4$ Pa) ou en surpression, la pression pouvant être comprise entre 1 et 4 bars.

Généralement, on préfère travailler sous une pression supérieure entre 1 et 4 bars et sous azote.

D'un point de vue pratique, le procédé selon l'invention est simple à mettre en oeuvre.

Un mode préféré de mise en oeuvre de ce procédé consiste à charger l'alcool aliphatique bicyclique **(I')** et à établir l'atmosphère de gaz inerte.

On commence par déshydrater le milieu réactionnel en éliminant les volatils par chauffage entre 120° C et 150° C.

On porte le milieu rèactionnel à la température désirée. entre 150° C et 200° C.

On introduit ensuite le réactif (**Wop et/ou Woe**) e. g : l'oxyde d'alkylène, sous atmosphère inerte ou sous pression réduite.

Dans le cas où l'on prépare un composé mixte, à la fois polyoxyéthyléné et polyoxypropyléné, on introduit, successivement, les oxydes d'alkylène de nature différente, pour obtenir une configuration en motifs séquencés. Lorsque l'on vise une configuration en motifs statistiques, tous les oxydes d'alkylène sont incorporés en même temps.

En fin de réaction. on procède à une étape de neutralisation. de façon à amener le pH du produit obtenu entre 5 et 8, de préférence entre 6 et 7.

On obtient ainsi le dérivé aliphatique **(I')** porteur d'un cycle polyoxyalkyléné. Le produit obtenu est sous forme liquide et/ou sous forme pâteuse.

Si le catalyseur mis en oeuvre est basique, la neutralisation est effectuée à l'aide d'un acide quelconque, de préférence à l'aide d'acide acétique.

Dans le cas d'un catalyseur acide, on neutralise à l'aide d'une base, de préférence à l'aide de soude, de carbonate ou de bicarbonate de sodium.

Si l'on souhaite débarrasser le produit obtenu du catalyseur, on le fait selon les techniques traditionnelles, notamment par un traitement à l'aide d'un réactif qui permet de rendre le catalyseur sous la forme d'un sel insoluble qui est alors éliminé selon les techniques classiques de séparation solide/liquide. notamment par filtration.

Il est à noter que le rapport molaire, entre les réactifs **(I')** et (**Wop et Woe**) et les paramètres p et q, est déterminé aisément par l'homme du métier en fonction du produit que l'on souhaite obtenir (notamment polymère séquencé ou statistique).

S'agissant des composés **(I")** précurseurs des composés **(Ib)**.[m = 0], $Z = Z_3$-, l'un de leurs procédés d'obtention consiste essentiellement en une condensation entre au moins un bicyclo[a.b.c.]-n-alc 1-en-yle (avec n = 1 à 6 et a + b + c = 5 ) et au moins un diol, ladite condensation s'effectuant avantageusement, en l'absence de solvant, et éventuellement en présence de catalyseur.

A titre d'exemple, on peut mentionner le cas de la préparation d'arbanol (ou isopropoxycamphane ou bien encore (isobornyloxy)2-isopropanol) selon un procédé de synthèse connu, par réaction de propylèneglycol sur le camphène, comme décrit dans la demande de brevet SANYO-JP-A-620 331 35 du 13 Février 1987 ou le brevet US-A-3 354 225. Pour obtenir **Ib** = I.1, l'arbanol est ensuite mis en présence de **Wop** = e.g. oxyde de propylène et de **Woe** = e.g. oxyde d'éthylène.

La fonctionnalisation facultative qui suit peut être, par exemple, une éthérification ou une estérification de l'hydrogène terminal en $R^6$ de la formule **(I)**. Cette étape s'opère de manière classique et connue en elle-même. Il est à noter que, dès que l'on procède à cette fonctionnalisation, il est préférable de réaliser au préalable la neutralisation.

A titre d'exemples, on évoque ci-après diverses possibilités non limitatives de fonctionnalisation de l'hydrogène terminal par un radical $R^6$.

- éther sulfates : $R_6 = SO_3M$ :
  Cela permet d'améliorer, de façon significative, les propriétés des produits, en particulier les pouvoirs mouillant et détergent. Les produits sont insensibles à la dureté de l'eau. Les produits sont moins irritants que les alkylsulfates correspondants.
  La synthèse s'effectue de façon conventionnelle par l'emploi de réactifs, tels que le trioxyde de soufre, l'acide sulfurique ou l'oléum, l'acide chlorosulfonique, ou l'acide sulfamique. De préférence, on utilisera les complexes du trioxyde de soufre, en particulier avec des bases de Lewis, comme la pyridine, le dioxanne et l'acide sulfamique.
  La réaction s'effectue dans des conditions connues de l'homme de l'art comme décrit dans PROCTER & GAMBLE, GB 1 111 208 (1968) et GAF, US 3 392 185 (1968). Les produits peuvent être obtenus sous forme de sels de

métaux alcalins ou d'ammonium, après neutralisation de l'hémiester sulfurique avec une base appropriée.

- éther phosphates : $R^6$ = $OPO_3M$ :
  Ces produits sont préparés lorsque de bonnes solubilités en milieu électrolyte sont recherchées.
  Cette propriété est importante, en particulier pour les applications détergence. Différentes méthodes de phosphatation peuvent être employées à l'aide de réactifs, comme l'acide phosphorique, l'anhydride phosphorique, l'oxychiorure de phosphore ou l'acide polyphosphorique, comme décrit dans GAF, US 3 331 896 (1967). Les produits ainsi obtenus présentent une grande stabilité chimique et sont particulièrement appréciés comme dispersants.
- éther carboxylates : $R^6$ = $(CH_2)_n$-COONa avec n = 1,2 :
  Ces produits présentent une bonne solubilitè en milieu caustique et une excellente stabilité aux électrolytes. Le pouvoir détergent à haute température est très augmenté et les produits présentent des propriétés antiredéposition, ce qui permet leurs applications en traitement textile.
  La préparation de ces produits peut s'effectuer de deux matueres :

  . pour n = 1, on met en présence le produit avec les sels appropriés de l'acide monochloroacétique en présence de soude à des températures voisines de 50-55° C, selon une procédure connue de l'homme de l'art, comme par exemple SANDOZ US 2 623 900,
  . pour n = 2, on utilise des dérivés de l'acide acrylique comme décrit dans ARCHER, US 2 983 738.

- éther sulfonates : $R_6$ = $(CH_2)_1$-$SO_3M$ avec 1 = 2,3.
  Cela permet d'augmenter la stabilité thermique des produits et la compatibilité avec les milieux salins permet des applications dans les domaines du pétrole et des travaux publics.

  . Pour 1 = 2, deux procédures de préparation sont envisageables :
  On prépare le dérivé chloré correspondant par traitement au chlorure de thionyle. Ce dérivé chloré est ensuite traité par une solution d'hydrogénosulfite alcalin selon ROHM & HAAS, US 2 115 192 (1938).
  On peut également préparer le produit par une procédure ne générant pas de sels, comme le traitement par le vinylsulfonate de sodium en présence d'hydroxydes de métaux alcalins selon STORK, US 4 978 780.
  . Pour 1 = 3, on procède à un traitement par la propane sulfone selon K. SUGA, Austr. J. Chem., 21, 2333 (1968).

- $R^6$ = éthers chlorés ou alkyles :
  En dehors de leur grande stabilité en milieu alcalin, ces dérivés sont particulièrement appréciés pour leur excellent pouvoir détergent au-dessus de leur température de trouble. Ces produits possèdent de faibles pouvoirs moussants, ce qui les destine à des applications du type nettoyage de bouteilles et détergence, en particulier pour le lavage du linge.
  S'agissant des éthers chlorés, les produits sont préparés par traitement au chlorure de thionyle en présence d'hydroxydes de métaux alcalins selon ROHM & HAAS, US 2 817 686.
  Quand $R^6$ = $C_1$-$C_4$ alkyle ou alkylphényle, les produits sont préparés par traitement par l'halogénure d'alkyle ou d'alkylphényle correspondant. On préfère utiliser comme halogénure d'alkylphényle, le chlorure de benzyle selon ROHM & HAAS, US 2 913 416.

**APPLICATION INDUSTRIELLE**

Dans d'autres de ses aspects. l'invention est relative aux applications des composés **(I)** **(Ia)**, **(Ib)** décrits supra.

C'est ainsi que l'invention a également pour objet une composition tensioactive comprenant au moins un de ces composés **(I)**.

Avantageusement, une telle composition tensioactive possède un pouvoir tensioactif ajustable par l'intermédiaire des paramétres p et q de la formule **(I)**.

La présente invention concerne donc également, mais non limitativement, des formulations détergentes contenant de cette composition tensioactive.

De telles formulations détergentes constituent des exemples concrets d'exploitation des caractéristiques avantageuses des composés **(I)**, à savoir :

- ces composés **(I)** sont issus d'une matière première naturelle renouvelable,
- ils sont peu toxiques et sans effets nocifs sur l'environnement,
- ils sont d'un coût de revient faible.
- et ils offrent des propriétés tensioactives remarquables, tout en n'entraînant pas la production de mousse et en ayant une odeur parfumée agréable, qui évite le recours à d'autres additifs parfumants classiques onéreux.

Les composés (I) peuvent être présents à raison d'environ 3 à 40 % en poids dans les formulations détergentes selon l'invention.

Par formulation détergente, on entend, non seulement toute formulation lessivielle, poudre ou liquide, destinée à un emploi, tant en machine à laver le linge, lave-vaisselle que pour les nettoyages ménagers en général, mais aussi toute formulation utilisable pour le nettoyage/dégraissage de surfaces métalliques et, plus généralement, de surfaces dures, e. g. plaques en acier.

D'autres additifs, du type de ceux décrits ci-après, peuvent être présents dans les formulations détergentes, à côté des produits d'origine terpénique de l'invention.

- *AGENTS TENSIO-ACTIFS* autres, en quantités correspondant à environ 3-40 % en poids par rapport à la composition détergente, agents tensio-actifs tels que :

  . AGENTS TENSIO-ACTIFS ANIONIQUES :

  * les alkylesters sulfonates de formule $R^{10}$-CH($SO_3$M)-COO$R^{11}$, où $R^{10}$ représente un radical alkyle en $C_8$-$C_{20}$, de préférence en $C_{10}$-$C_{16}$, $R^{11}$ un radical alkyle en $C_1$-$C_6$, de préférence en $C_1$-$C_3$, et M un cation alcalin (sodium, potassium, lithium), ammonium substitué ou non substitué (méthyl-, diméthyl-, triméthyl-, tetraméthylammonium, diméthylpiperidinium ...) ou dérivé d'une alcanolamine (monoéthanolamine, diéthanolamine, triéthanolamine ...). On peut citer tout particulièrement les méthyl ester sulfonates dont le radical $R^{10}$ est en $C_{14}$-$C_{16}$ ;
  * les alkylsulfates de formule $R^{12}OSO_3M$, où $R^{12}$ représente un radical alkyle ou hydroxyalkyle en $C_{10}$-$C_{24}$, de préférence en $C_{12}$-$C_{20}$, et tout particulièrement en $C_{12}$-$C_{18}$, M représentant un atome d'hydrogène ou un cation de même définition que ci-dessus. ainsi que leurs dérivés éthoxylénés (OE) et/ou propoxylénés (OP), présentant en moyenne de 0,5 à 6 motifs, de préférence de 0.5 à 3 motifs OE et/ou OP;
  * les alkylamides sulfates de formule $R^{13}CONHR^{14}OSO_3M$ où $R^{13}$ représente un radical alkyle en $C_2$-$C_{22}$, de préférence en $C_6$-$C_{20}$, $R^{14}$ un radical alkyle en $C_2$-$C_3$, M représentant un atome d'hydrogène ou un cation de même définition que ci-dessus, ainsi que leurs dérivés éthoxylénés (OE) et/ou propoxylénés (OP), présentant en moyenne de 0,5 à 60 motifs OE et/ou OP ;
  * les sels d'acides gras saturés ou insaturés en $C_8$-$C_{24}$, de préférence en $C_{14}$-$C_{20}$, les alkylbenzènesulfonates en $C_9$-$C_{20}$, les alkylsulfonates primaires ou secondaires en $C_8$-$C_{22}$, les allrylglycérol sulfonates, les acides polycarboxyliques sulfonés décrits dans GB-A-1 082 179, les sulfonates de paraffine, les N-acyl N-alkyllaurates, les alkylphosphates, les iséthionates, les alkylsuccinamates les alkylsulfosuccinates, les monoesters ou diesters de sulfosuccinates. les N-acyl sarcosinates, les sulfates d'alkylglycosides, les polyéthoxycarboxylates, le cation étant un métal alcalin (sodium, potassium. lithium), un reste ammonium substitué ou non substitué (méthyl-, diméthyl-, triméthyl-, tetraméthylammonium, diméthylpiperidinium ...) ou dérivé d'une alcanolamine (monoéthanolamine, diéthanolamine, triéthanolamine...) ;

  . AGENTS TENSIO-ACTIFS NON-IONIQUES :

  * les alkylphénols polyoxyalkylénés (polyoxyéthylénés, polyoxypropylénés, polyoxybutylénés) dont le substituant alkyle est en $C_6$-$C_{12}$ et contenant de 5 à 25 motifs oxyalkylènes à titre d'exemple. on peut citer les TRITON X-45, X-114, X-100 ou X-102 commercialisés par ROHM & HAAS Cy.;
  * les glucosamides, glucamides, glycérolamides ;
  * les alcools aliphatiques en $C_8$-$C_{22}$ polyoxyalkylénés contenant de 1 à 25 motifs oxyalkylènes (oxyéthylène, oxypropylène) ; à titre d'exemple, on peut citer les TERGITOL 15-S-9, TERGITOL 24-L-6 NMW commercialisés par UNION CARBIDE Corp., NEODOL 45-9, NEODOL 23-65. NEODOL 45-7, NEODOL 45-4 commercialisés par SHELL CHEMICAL Cy., KYRO EOB commercialisé par THE PROCTER & GAMBLE Cie.
  * les produits résultant de la condensation de l'oxyde d'éthylène, le composé résultant de la condensation de l'oxyde de propylène avec le propylène glycol, tels les PLURONIC commercialisés par BASF
  * les produits résultant de la condensation de l'oxyde d'éthylène, le composé résultant de la condensation de l'oxyde de propylène avec l'éthylènediamine, tels les TETRONIC commercialisés par BASF;
  * les oxydes d'amines, tels que les oxydes d'alkyl $C_{10}$-$C_{18}$ diméthylamines, les oxydes d'alkoxy $C_8$-$C_{22}$ éthyl dihydroxy éthylamines
  * les alkylpolyglycosides décrits dans US-A-4 565 647;
  * les amides d'acides gras en $C_8$-$C_{20}$ ;
  * les acides gras éthoxylés,
  * les amides gras éthorylés.

* les amines éthoxylées.

. AGENTS TENSIO-ACTIFS CATIONIQUES :

* les halogénures d'allcyldiméthylammonium,

. AGENTS TENSIO-ACTIFS AMPHOTERES ET ZWITTERIONIQUES :

* les alkyldiméthylbétaïnes, les alkylamidopropyldiméthylbétaïnes, les alkyltriméthylsulfobétaïnes, les produits de condensation d'acides gras et d'hydrolysats de proteines.

- *AGENTS BUILDERS*, en quantités correspondant à environ 5-50 % de preférence à environ 5-30 % en poids pour les formules détergentes liquides. ou à environ 10-80 %, de preférence 15-50 % en poids pour les formules détergentes en poudres, agents builders tels que :

. BUILDERS INORGANIQUES :

* les polyphosphates (tripolyphosphates, pyrophosphates, orthophosphates, hexamétaphosphates) de métaux alcalins, d'ammonium ou d'alcanolamines;
* les tetraborates ou les précurseurs de borates;
* les silicates, en particulier ceux présentant un rapport $SiO_2/Na_2O$ de l'ordre de 1,6/1 à 3,2/1 et les silicates lamellaires décrits dans US-A-4 664 839 ;
* les carbonates (bicarbonates, sesquicarbonates) alcalins ou alcalinoterreux ;
* les cogranulés de silicates hydratés de métaux alcalins et de carbonates de métaux alcalins (sodium ou de potassium) riches en atomes de silicium sous forme Q2 ou Q3, décrits dans EP-A 488 868;
* les aluminosilicates cristallins ou amorphes de métaux alcalins (sodium, potassium) ou d'ammonium, tels que les zéolithes A, P, X ... ; la zéolithe A de taille de particules de l'ordre de 0,1-10 micromètres est préférée;

. BUILDERS ORGANIQUES :

* les polyphosphonates hydrosolubles (éthane 1-hydroxy- 1, 1 - diphosphonates, sels de mèthylène diphosphonates...);
* les polymères ou de copolymères carboxyliques ou leurs sels hydrosolubles tels que :

  .. les éthers polycarboxylates (acide oxydisuccinique et ses sels, acide monosuccinique/tartrique et ses sels. acide disuccinique/tartrique et ses sels),
  .. les éthers hydroxypolycarboxylates,
  .. l'acide citrique et ses sels, l'acide mellitique, l'acide succinique et leurs sels,
  .. les sels d'acides polyacétiques (éthylènediaminetetraacétates, nitrilotriacétates, N-(2 hydroxyéthyl)-nitrilodiacétates),
  .. les acides alkyl C5-C20 succiniques et leurs sels (2-dodécénylsuccinates, lauryl succinates),
  .. les esters polyacétals carboxyliques,
  .. l'acide polyaspartique, l'acide polyglutamique et leurs sels;

* les polyimides dérivés de la polycondensation de l'acide aspartique et/ou de l'acide glutamique;
* les dérivés polycarboxyméthylés de l'acide glutamique ou d'autres acides aminés.

- *AGENTS DE BLANCHIMENT*, en quantités d'environ 0,1-20 %, de préférence environ 1-10 % en poids, éventuellement associés à des *ACTIVATEURS DE BLANCHIMENT,* en quantités d'environ 0,1-60 %, de préférence d'environ 0,5-40 % en poids, agents et activateurs tels que :

. AGENTS DE BLANCHIMENT.

* les perborates, tels que le perborate de sodium monohydraté ou tétrahydraté :
* les composés peroxygénés, tels que le carbonate de sodium peroxyhydraté, le pyrophosphate peroxyhydraté, l'urée peroxyhydratée. le peroxyde de sodium, le persulfate de sodium, de préférence associés à un activateur de blanchiment générant, in situ, dans le milieu lessiviel, un peroxyacide carboxylique parmi ces

activateurs, on peut mentionner, la tétraacetyléthylène diamine, la tetraacétyl méthylène diamine, le tetraacétyl glycoluril, le p-acétoxybenzéne sulfonate de sodium, le pentaacétyl glucose, l'octaacétyl lactose...

* les acides percarboxyliques et leurs sels (appelés "percarbonates"), tels que le monoperoxyphtalate de magnésium hexahydraté, le métachloroperbenzoate de magnésium, l'acide 4-nonylamino-4-oxoperoxybutyrique, l'acide 6-nonylamino-6-oxoperoxvcaproique, l'acide diperoxydodécanedioique, le nonylamide de l'acide peroxysuccinique, l'acide décyldiperoxysuccinique.

Ces agents peuvent être associés à au moins un des agents anti-salissures ou antiredéposition mentionnés ci-après. Peuvent également être mentionnés des agents de blanchiment non oxygénés, agissant par photoactivation en présence d'oxygène, agents tels que les phtalocyanines d'aluminium et/ou de zinc sulfonées.

- *AGENTS ANTI-SALISSURES*, en quantités de l'ordre de 0,01-10 %, de préférence environ 0,1-5% et, tout particulièrement, de l'ordre de 0,2-3 % en poids, agents tels que :

  . les dérivés cellulosiques, tels que les hydroxyéthers de cellulose, la méthylcellulose, l'éthylcellulose, l'hydroxypropyl méthylcellulose, l'hydroxybutyl méthylcellulose;
  . les polyvinylesters greffés sur des troncs polyalkylènes, tels que les polyvinylacétates greffés sur des troncs polyoxyéthylènes (EP-A-219 048);
  . les alcools polyvinyliques,
  . les copolymères polyesters à base de motifs ethylène téréphtalate et/ou propylène téréphtalate et polyoxyéthylène téréphtalate, avec un rapport molaire (nombre de motifs) ethylène téréphtalate et/ou propylène téréphalate / (nombre de motifs) polyoxyéthylène téréphtalate de l'ordre de 1/10 à 10/1, de préférence de l'ordre de 1/1 à 9/1, les polyoxyéthylène téréphtalates présentant des unités polyoxyéthylène ayant un poids moléculaire de l'ordre de 300 à 5000, de préférence de l'ordre de 600 à 5000 (US-A-3 959 230, US-A-3 893 929, US-A-4 116 896, US-A-4 702 857, US-A-4 770 666);
  . les oligomères polyesters sulfonés obtenus par sulfonation d'un oligomère dérivé de l'alcool allylique éthoxylé, du diméthyltéréphtalate et du 1,2 propylène diol, présentant de 1 à 4 groupes sulfonés (US-A-4 968 451);
  . les copolymères polyesters à base de motifs propylène téréphtalate et polyoxyéthylène téréphtalate et terminés par des motifs éthyles, méthyles (US-A-4 711 730) ou des oligomères polyesters terminés par des groupes alkylpolyéthoxy (US-A-4 702 857) ou des groupes anioniques sulfopolyéthoxy (US-A-4 721 580), sulfoaroyles (US-A-4 877 896);
  . les polyesters-polyuréthanes obtenus par réaction d'un polyester de masse moléculaire en nombre de 300-4 000 obtenu à partir d'acide adipique et/ou d'acide téréphtalique et/ou d'acide sulfoisophtalique et d'un diol de masse inférieure à 300, sur un prépolymère à groupements isocyanates terminaux obtenu à partir d'un polyoxyéthylène glycol de masse moléculaire de 600-4 000 et d'un diisocyanate (FR-A-2 334 698).

- *AGENTS ANTI-REDEPOSITION*, en quantités d'environ 0,01-10 % en poids pour une composition détergente en poudre, d'environ 0,01-5%en poids pour une composition détergente liquide, agents tels que :

  . les monoamines ou polyamines éthoxylées, les polymères d'amines éthoxylées (US-A-4 597 898, EP-A-11 984);
  . la carboxyméthylcellulose;
  . les oligomères polyesters sulfones obtenus par condensation de l'acide isophtalique, du sulfosuccinate de diméthyle et de diéthylène glycol (FR-A-2 236 926);
  . les polyvinylpyrrolidones.

- *AGENTS CHELATANTS* du fer et du magnésium, en quantités de l'ordre de 0,1 - 10 %, de préférence de l'ordre de 0,1-3 % en poids, agents tels que :

  . les arninocarboxylates, tels que les éthylènediaminetétraacétates, hydroxyéthyl éthylènediaminetriacétates, nitrilotriacétates;
  . les aminophosphonates tels que les nitrilotris(méthylène phosphonates);
  . les composés aromatiques polyfonctionnels, tels que les dihydroxydisulfobenzènes.

- *AGENTS DISPERSANTS POLYMERIQUES*, en quantité de l'ordre de 0,1-7 % en poids. pour contrôler la dureté en calcium et magnésium, agents tels que :

. les sels hydrosolubles d'acides polycarboxyliques de masse moléculaire de l'ordre de 2 000 à 100 000, obtenus par polymérisation ou copolymérisation d'acides carboxyliques éthyléniquement insaturés tels que acide acrylique, acide ou anhydride maleique, acide fumarique, acide itaconique, acide aconitique, acide mesaconique, acide citraconique, acide méthylènemalonique et, tout particulièrement, les polyacrylates de masse moléculaire de l'ordre de 2 000 à 10 000 (US-A-3 308 067), les copolymères d'acide acrylique et d'anhydride maléique de masse moléculaire de l'ordre de 5 000 à 75 000 (EP-A-66 915);
. les polyéthylèneglycols de masse moléculaire de l'ordre de 1 000 à 50 000

- *AGENTS DE FLUORESCENCE (BRIGHTENERS)*, en quantité d'environ 0,05-1,2% en poids, agents tels que :

. les dérivés de stilbène, pyrazoline, coumarine, acide fumarique, acide cinnamique, azoles, methinecyanines, thiophènes ... ("The production and application of fluorescent brightening agents" - M. ZAHARADNIK, publié par John Wiley & Sons, New York, 1982).

- *AGENTS SUPPRESSEURS DE MOUSSES*, en quantités pouvant aller jusqu'à 5% en poids, agents tels que :

. les acides gras monocarboxyliques en $C_{10}$-$C_{24}$ ou leurs sels alcalins, d'ammonium ou alcanolamines, les triglycérides d'acides gras;
. les hydrocarbures saturés ou insaturés aliphatiques, alicycliques, aromatiques ou hétèrocycliques, tels que les paraffines, les cires ;
. les N-alkylaminotriazines;
. les monostéarylphosphates, les monostéaryl alcool phosphates;
. les huiles ou résines polyorganosiloxanes, éventuellement combinées avec des particules de silice.

- *AGENTS ADOUCISSANTS*, en quantités d'environ 0,5-10 % en poids, agents tels que les argiles.
- *ENZYMES* en quantité pouvant aller jusqu'à 5 mg en poids, de préférence de l'ordre de 0,05-3 mg d'enzyme active/g de composition détergente, enzymes telles que :

. les protéases, amylases, lipases, cellulases, peroxydases (US-A-3 553 139, US-A-4 101 457, US-A-4 507 219, US-A-4 261 868).

- *AUTRES ADDITIFS*, tels que :

. des alcools (méthanol, éthanol, propanol, isopropanol, propanediol, éthylène glycol, glycérine);
. des agents tampons;
. des parfums;
. des pigments.

Dans le domaine de la détergence ménagère, la présente invention vise plus particulièrement les compositions pour liquides de lavage de la vaisselle à la main.

Lesdites compositions contiennent de l'ordre de 0,1 à 20%, de préférence de l'ordre de 0,5 à 10% de leur poids de dérivés d'origine terpénique faisant l'objet de l'invention et de l'ordre de 0,5 à 40%, de préférence de l'ordre de 1 à 20% de leur poids d'au moins un agent tensio-actif anionique ou non-ionique.

Les dérivés d'origine terpénique faisant l'objet de l'invention préférentiels sont ceux déjà mentionnés ci-dessus.

Parmi les agents tensio-actifs anioniques pouvant être présents, on peut notamment citer les alkylsulfates, les alkylethersulfates, les alkylsulfonates, les alkylbenzènesulfonates, les savons, les alkyléthercarboxylates, les N-acylsarcosinates, les alkyliséthionates, les N-acyl N-alkyltaurates, les alkylphosphates, les alkylsulfosuccinates, les alkylsulfosuccinamates, les dérivés sulfonés d'acides gras ...

Parmi les agents tensio-actifs non-ioniques pouvant être présents, on peut notamment citer les oxydes d'amines, les alkylglucamides, les alkylpolyglycosides, les dérivés oxyalkylénés d'alcools gras comme les PLANTAREN® commercialisé par

A côté des dérivés d'origine terpénique faisant l'objet de l'invention et des agents tensio-actifs anioniques pouvent être présents dans les compositions pour liquides de lavage de la vaisselle à la main, d'autres additifs tels que

. des agents tensio-actifs amphotères et zwitterioniques comme les alkyldiméthylbétaines, les alkylamidopropylbétaines, les alkylsultaines, les alkyl amphoacétates et diacétates, les produits de condensation d'acides gras sur les protéines ou les hydrolysats de protéines, les dérivés amphotères des alkylpolyamines comme l'AMPHIONIC XL® commercialisé par RHONE-POULENC, AMPHOLAC 7T/X® et AMPHOLAC 7C/X® commercialisés par BEROL

NOBEL

. des agents bactéricides ou désinfectants comme le triclosan

. des polymères cationiques synthétiques comme le MIRAPOL 550®, le MIRAPOL A15® commercialisés par RHONE-POULENC, le MERQUAT 550® commercialisé par CALGON ...

. les polymères utilisés pour contrôler la viscosité du mélange et/ou la stabilité des mousses formées à l'utilisation, comme les dérivés de cellulose ou de guar (carboxyméthylcellulose, hydroxyéthylcellulose, hydroxypropylguar, carboxyméthylguar, carboxyméthylhydroxypropylguar ...)

. des agents hydrotropes, comme les alcools courts en $C_2$-$C_8$, en particulier l'éthanol, les diols et glycols comme le diéthylène glycol, dipropylèneglycol, ...

. des agents hydratants ou humectants pour la peau comme le glycérol, l'urée ou des agents protecteurs de la peau, comme les protéines ou hydrolysats de proteines, les polymères cationiques comme les dérivés cationiques du guar (JAGUAR C13S®, JAGUAR C162$^6$, HICARE 1000® commercialisés par RHONE-POULENC,

. des colorants, des parfums, des conservateurs ...

Les dérivés d'origine terpénique faisant l'objet de l'invention peuvent également entrer dans des compositions cosmétiques.

On entend par le terme composition ou formulation cosmétique tous les produits ou préparations cosmétiques comme celles décrites dans l'annexe I ("Illustrative list by category of cosmetic products") de la directive européenne n° 76/768/CEE du 27 juillet 1976 dite "directive cosmétique"

Les dérivés d'origine terpénique faisant l'objet de l'invention peuvent être utilisées plus particulièrement dans des formulations de lotions, laits de toilettes, compositions démaquillantes, crèmes de soins ou crèmes ou lotions de protection contre le soleil et le rayonnement ultra-violet, mousses gels coiffants ou toute formulation utilisée pour le coiffage ou pour faciliter le peignage des cheveux, shampoings pour cheveux ou le corps, gels de nettoyage du visage ou du corps, savons liquides, compositions moussantes pour le bain, formulations utilisées pour le nettoyage des dents ou de la cavité buccale comme les bains de bouche ou les dentifrices. Lesdits dérivés d'origine terpénique faisant l'objet de l'invention peuvent aussi être utilisées seules ou en association dans la formulation de savons ou de pains de toilette Ils peuvent aussi être mis en oeuvre pour la confection de formulations de rinçage ou de baumes conditionneurs pours les cheveux ou la peau.

Les compositions cosmétiques contiennent généralement des agents tensioactifs qui servent à disperser, émulsionner, solubiliser, stabiliser divers composés utilisés pour leurs propriétés émollientes ou humectantes. Les agents tensioactifs sont utilisés dans ces compositions à des concentrations variant de 0,05 à 10% en poids de la préparation.

Parmi les agents humectants, on peut citer le glycérol, le sorbitol, l'urée, le collagène, la gélatine, l'aloe vera, l'acide hyaluronique, ...

Les émollients sont généralement choisis parmi les alkylmonoglycérides, les alkyldiglycérides, les triglycérides comme les huiles extraites des plantes et des végétaux (huiles de palme, de coprah, de graine de coton, de soja, de tournesol, d'olive, de pépin de raisin, de sésame, d'arachide, de ricin...) ou les huiles d'origine animale (suif, huiles de poisson,...), des dérivés de ces huiles comme les huiles hydrogénées, les dérivés de la lanoline, les huiles minérales ou les huiles paraffiniques, le perhydrosqualane, le squalène, les diols comme le 1-2-propanediol, le 1-3-butanediol, l'alcool cétylique, l'alcool stéarylique, l'alcool oléique, les polyéthylèneglycols ou polypropylèneglycols, les esters gras comme le palmitate d'isopropyl, le cocoate d'éthyl-2-hexyl, le myristyl myristate, les esters de l'acide lactique, l'acide stéarique, l'acide béhennique, l'acide isostéarique, les huiles silicones regroupant les polydiméthylsiloxanes cycliques, les polydiméthylsiloxanes a-w hydroxylées, les polydiméthylsiloxanes a-w triméthylsilyllés, les polyorganosiloxanes comme les polyalkylméthylsiloxanes, les polyméthylphénylsiloxanes, les polydiphénylsiloxanes, les dérivés aminés des silicones, les cires silicones, les silicones copolyéthers (comme l'huile SILBIONE 70646® commercialisée par la société RHONE-POLLENC ou DC 190® commercialisée par DOW CORNING) ou les dérivés mixtes de silicones comme les copolymères mixtes polyalkylméthylsiloxanessilicones copolyéthers.

A ces composés, on peut ajouter des poudres ou des particules minérales comme du carbonate de calcium, des oxydes minéraux sous forme de poudre ou sous forme colloidale (particules de taille inférieure ou de l'ordre de un micromètre, parfois de quelque dizaines de nanomètres) comme du dioxyde de titane, de la silice, des sels d'aluminium utilisés généralement comme antitranspirants, du kaolin, du talc, des argiles et leurs dérivés, ...

Des agents conservateurs comme les méthyl, éthyl, propyl et butyl esters de l'acide p-hydroxybenzoïque, le benzoate de sodium, le GERMABEN® ou tout agent chimique évitant la prolifération bactérienne ou des moississures et utilisé traditionnellement dans les compositions cosmétiques sont généralement introduits dans ces compositions à hauteur de 0,01 à 3% en poids.

Alternativement à ces agents chimiques, on peut parfois utiliser des agents modifiants l'activité de l'eau et augmentant fortement la pression osmotique comme les carbohydrates ou des sels.

Pour protéger la peau ou les cheveux des agressions du soleil et des rayons UV, on peut ajouter à ces formulations des filtres solaires qui sont, soit des composés chimiques absorbant fortement le rayonnement UV comme les composés autorisés dans la directive Européenne N° 76/768/CEE, ses annexes et les modifications ultérieures de cette directive, soit le dioxyde de titane ou les oxydes de cérium sous forme de poudre ou de particules colloïdales.

Des parfums, des colorants ou des pigments peuvent également être ajoutés.

Peuvent aussi être présents des polymères viscosants ou gélifiants (comme les polyacrylates réticulés CARBOPOL® commercialisés par GOODRICH-, les dérivés de la cellulose comme l'hydroxypropylcellulose, la carboxyméthylcellulose, les guars et leurs dérivés, la caroube, la gomme de tara ou de cassia, la gomme xanthane, les alginates, les carraghénannes, les dérivés de la chitine comme le chitosan ...

Un dentifrice peut contenir, outre des agents abrasif's comme de la silice ou du carbonate de calcium (utilisés à 5 à 25% en poids du total de la composition), de 0.1 à 5% en poids desdits tensioactifs de l'invention, pris seuls ou en mélange avec les tensio-actifs usuels tels que des tensioactifs anioniques, non ioniques ou zwitterioriques comme les alkylbétaines ou alkylamidopropylbétaines ou les amphotères ou des associations de ces composés. Ces agents tensioactifs qui sont utilisés comme agents moussants lors de l'utilisation de la composition, ainsi que pour leur pouvoir nettoyant, désinfectant. Parfois, ils sont utilisés spécifiquement pour une action "thérapeutique" comme les alkyls sarcosinates qui protègent les dents en inhibant l'activité enzymatique des bactéries responsables des infections des dents ou des genclves.

Les compositions de dentifrices comportent aussi de 5 à 85% d'agents dits humectants comme le glycérol, le sorbitol, les polyéthylèneglycols, le lactilol, le xylitol.

Le comportement rhéologique de la pâte dentaire, c'est-à dire sa viscosité, sa tenue sur la brosse, la facilité de son extrusion à la sortie du tube ou du dispenseur, le caractère non filant de la pâte est contrôlé par des agents épaississants comme certaines silice utilisées à cet effet (TIXOSIL 43® commercialisées par RHONE-POULENC) et/ou des polymères utilisés seuls ou en association comme la gomme Xanthane, la gomme guar, les dérivés de la cellulose (Carboxyméthylcellulose par exemple), des polyacrylates réticulés comme les CARBOPOL® distribués par GOODRICH, des alginates ou des carraghénannes, de la VISCARIN ® Le total des agents épaississants représente de 0.1 à 15 % en poids de la composition d'un dentifrice.

On retrouve aussi généralement associés à ces divers constituants des agents thérapeutiques comme certains sels de fluor ou de potassium, des arômes, des agents édulcorants et de l'eau.

Les dérivés d'origine terpénique faisant l'objet de l'invention peuvent être aussi utilisées dans des formulations de pains de toilettes appelées savonnettes ou savons.

Les compositions classiques de pains de toilette comprennent généralement des sels d'acide gras utilisés en association avec des tensioactifs de l'invention et éventuellement des tensio-actifs autres que les sels d'acides gras ou les acides gras eux-mêmes. Ces compositions peuvent même ne contenir aucun acide gras ou sel d'acide gras et leurs formulations reposent alors sur d'autres tensioactif's comme par exemple les alkyl isethionates de sodium en $C_8$-$C_{22}$ ou les alkylsulfates de sodium en $C_8$-$C_{22}$.

A ces compositions on peut aussi ajouter différents constituants utiles pour diminuer l'irritation ou l'aggression de la peau comme les sels de métaux alcalins ou les isethionates ou pour favoriser l'hydratation de la peau comme certains carbohydrates (glycerol, sorbitol par exemple), des polyethylènes glycol ou polypropylène glycol, des dérivés alcoxylés des sucres ou de leurs dérivés (méthyl glucose par exemple), des polymères hydrosolubles ou hydrodispersables comme le collagène ou certains dérivés non allergisants de protéines animales ou végétales (hydrolysats de protéines de blé par exemple), des hydrocolloides naturels (gomme de guar, de caroube, de tara ...) ou issus de procédés de fermentation comme la gomme xanthane et les dérivés de ces polycarbohydrates comme les celluloses modifiées (par exemple Hydroxyéthylcellulose, carboxyméthylcellulose, celluloses cationiques comme les POLYMER JR® commercialisés par la société UNION CARBIDE), les dérivés du guar ou de la caroube comme leurs dérivés cationiques (JAGUAR C13S[6], JAGUAR C162® commercialisés par RHONE-POULENC) ou des dérivés non-ioniques (par exemple hydroxypropylguar), les dérivés anioniques (carboxyméthylguar) ou les dérivés mixtes non-ioniques/anioniques comme les carboxy-hydroxypropyl-guars ou non-ioniques/cationiques. On peut aussi ajouter alternativement ou en association des polymères synthétiques comme les polyacrylates ou des polymères cationiques synthétiques, connus sous le nom générique CTFA de "Polyquaternium", par exemple les polyméres MIRAPOL A15® ou MIRAPOL 550® de la société RHONE-POULENC.

On peut aussi avantageusement ajouter à ces compositions des agents séquestrants des métaux, plus particulièrement ceux sequestrants du calcium comme les ions citrates ou des agents émollients comme les silicones ou des huiles ou corps gras utilisés à ce propos dans l'industrie cosmétique (huiles minérales, esters d'acides gras, triglycérides, silicones, ....).

A ces ingrédients on rajoute généralement un ou des parfums, des colorants et/ou des agents opacifiants comme des pigments (particules d'oxyde de titane). On peut aussi incorporer dans la composition des agents bactéricides ou fongicides afin d'améliorer la désinfection de la peau.

Dans un pain de toilette dont la formulation est constituée principalement de savons d'acides gras monocarboxyliques (sels de sodium, potassium, mono-, di- ou tri- éthanolammonium); les teneurs en savons d'acides gras sont généralement de plus de 25% en poids de la formulation, généralement de 30 à 95% en poids.

Dans un pain de toilette dont la formulation repose sur d'autres constituants principaux que les savons d'acides gras, on trouve dans la formulation de 0 à 50 % en poids, préférentiellement de 1 à 40 % en poids de ces savons d'acides gras.

Ces compositions de pains de toilettes peuvent aussi contenir de 0 à 95%, de préférence de 0 à 60% d'agents tensioactifs autres que des savons, notamment les $C_8$-$C_{22}$ alkyl ou alkényl iséthionates,de même que les alkyléthersulfates, les alkylbétàines, les alkylamidopropylbétaïnes, les alkylampho-acétates, -diacétates, - propionates ou - dipropionates utilisés pour diminuer l'irritation pouvant être provoquée par les autres agents tensioactifs, principalement les agents tensioactifs anioniques.

De 1 à 15% d'acides gras libres en $C_8$-$C_{22}$ peuvent aussi être introduits dans les compositions de savons en tant qu'agents surgraissants ou pour modifier l'aspect et le caractère crèmeux de la mousse lors du lavage.

On peut aussi retrouver dans ces compositions des cires comme des cires paraffiniques, des cires naturelles comme la cire d'abeille ou l'ozokérite ou des cires silicones. Ces cires sont avantageusement utilisées pour améliorer l'aspect, la tenue, la processabilité et la conservation au stockage des pains de toilettes.

Les shampoings, et d'une manière plus générale les compositions détergentes pour l'usage personnel peuvent contenir, outre les dérivés d'origine terpénique faisant l'objet de l'invention, les additifs habituels présents dans ces types de formulation.

On peut citer en particulier

- des agents tensio-actifs anioniques, tels que les sels hydrosolubles d'alkylsulfates, d'alkyléthersulfates, les alkylisethionates et les alkyltaurates ou leurs sels, les alkylcarboxylates, les alkylsullosuccinates ou les alkylsuccinamates, les alkylsarcosinates, les dérivés alkyllés d'hydrolysats de protèines, les acylaspartates
- des agents tensio-actifs zwitterioniques ou amphotères, comme les alkylbétaines, les alkylamidopropylbétaines, les dérivés d'imidazoline tels que les alkylamphoacétates, alkylamphodiacétates, alkylamphopropionates, alkylamphodipropionates, les alkylsultaines ou les alkylamidopropylhydroxysultaines
- des agents tensio-actifs non-ioniques, comme les dérivés polyoxyéthylénés d'alcools aliphatiques ou arylaliphatiques en $C_8$-$C_{22}$, les alkylpolysaccharides présentant un groupement hydrophobe en $C_6$-$C_{30}$, de préférence en $C_{10}$-$C_{16}$ et un groupement polysaccharide, par exemple polyglycoside, comme groupement hydrophile ainsi que de 1 à 3 unités sucre, les dérivés alkylés d'aminosucres, comme les alkylglucamides produits par la réaction d'amidification d'un acide gras sur la N-méthylglucamine
- des amides dérivés d'acides gras utilisés généralement pour leur aptitude à améliorer le moussage des compositions ou à augmenter la viscosité desdites compositions; ces amides sont choisis généralement
- des agents conditionneurs (améliorant la peignabilité, le coiffage, le toucher et le volume de la chevelure) cationiques tels que les polymères cationiques synthétiques du type MIRAPOL AD1® ou MIRAPOL A550® commercialisés par RHONE-POULENC, des polymères cationiques naturels comme les dérivés cationiques du guar (JAGUAR C135®, JAGUAR C162® commercialisés par RHONE-POULENC) ou les dérivés cationiques de la cellulose (POLYMER JR400® commercialisé par UNION CARBIDE).
- des organopolysiloxanes utilisés tels quels ou en solution dans un de leurs solvants usuels (huiles silicones de basses masses, huiles paraffiniques très ramifiées, les esters gras par exemple du type palmitate d'isopropyle, ...), comme agents conditionneurs ou de brillance.
- des agents antipelliculaires, comme les pyridinesthiones, plus spécialement la zinc pyridinethione, les composés à base de sélénium comme le sulture de sélénium ou l'OCTOPYROx°, commercialisé par HOECHST
- des agents anti-parasites (anti-poux), comme le LINDANE ou différentes pyréthrines utilisées à ce propos.

Ces compositions peuvent aussi contenir des agents modifiant l'aspect ou la viscosité des formulations comme les composés perlescents à base de stéarate de polyéthylène glycol ou des polymères améliorant la viscosté ou la stabilité comme les CARBOPOL® commercialisés par GOODRICH, les hydrocolloides et leurs dérivés comme le le guar ou les guars modifiés, la caroube, la gomme xanthane, les dérivés de la cellulose (hydroxyéthylcellulose, marboxyméthylcellulose).

Les compositions classiques de shampoings contenant les dérivés d'origine terpénique faisant l'objet de l'invention peuvent être constituées de (pourcentages en poids)

| | | |
|---|---|---|
| . dérivés d'origine terpénique faisant l'objet de l'invention | | 1-15% |
| . agent tensio-actif anionique | | 0-15 % |
| . agent tensio-actif amphotère | | 0-10 % |
| . alcanolamide d'acide gras | | 0-5 % |
| . agent épaississant | | 0-5 % |
| . agent conditionneur | | 0-3 % |
| . parfum | | 0-2 % |
| . conservateur | | 0-2 % |
| . agent anipelliculaire | | 0-2 % |
| . eau | complément à | 100 % |

Les produits d'origine terpénique de l'invention peuvent, en outre, être utilisés comme agents de nettoyage industriel de surfaces métalliques.

Par ailleurs, on a vu que les composés de formule (I) exhalent une gamme d'odeurs variées très intéressantes.

Ainsi, e. g, les performances olfactives des dérivés du NOPOL ($I_a$) définis ci-dessus sont évaluées sur des solutions des produits dans l'éthanol ou le diéthylphtalate (10 % poids).

| p | q | FAMILLE | TENUE | DESCRIPTION OLFACTIVE |
|---|---|---|---|---|
| 0 | 3,3 | Citralée | tête/coeur | boisé, frais, épicée, gingembre |
| 2 | 0,0 | Hespéridée | tête/coeur | très frais, citron, thym, serpolet |
| 2 | 3,3 | Hespéridée | tête/coeur | très cireux, très boisé, gras |
| 2 | 5,1 | Hespéridée | tête | citronnée, très frais, bois, eau de Cologne |
| 2 | 7,5 | Hespéridée | tête/coeur | citronnée, cire, boisé |
| 2 | 10,5 | Hespéridée | tête/coeur | piquant, citronné, baies des bois |

Les rapports respectifs des paramètres p et q des radicaux **(Wop)** et **(Woe)** de la formule **(I)** permettent d'ajuster la performance olfactive à l'application recherchée.

Le grand avantage des produits **(I)** selon l'invention par rapport aux composants parfum conventionnels est que ces produits **(I)** sont tous solubles dans l'eau, ce qui simplifie grandement leur formulation, sans nécessiter l'emploi de solvants organiques.

Compte tenu de leurs performances olfactives, les produits de l'invention peuvent être utilisés comme ingrédients parfumants dans les compositions partumantes, substances et produits parfumés.

Par "compositions parfumantes", on désigne des mélanges de divers ingrédients, tels que solvants, supports solides ou liquides, fixateurs, composés odorants divers, etc..., dans lesquels sont incorporés au moins un des composés de formule **(I)**. Ces derniers sont utilisés pour procurer, à divers types de produits finis, la fragrance recherchée.

Les bases pour parfum constituent des exemples préférés de compositions parfumantes dans lesquelles les composes de formule **(I)** peuvent être avantageusement utilisés.

Les eaux de toilette, les lotions après rasage, les parfums, les savons, les gels de bain ou de douche ou les produits déodorants ou antiperspirants, qu'ils soient sous forme de sticks ou de lotions, constituent des exemples de substances ou de produits finis dans lesquels les composés de formule **(I)** apportent leur note originale.

Ils peuvent intervenir aussi dans les shampoings et dans les produits capillaires de tout type.

Ils peuvent aussi parfumer les talcs ou poudres de toute nature.

Ils peuvent également convenir pour les désodorisants d'air ambiant ou tout produit d'entretien.

La teneur des compositions parfumantes selon l'invention en composé de formule **(I)**, exprimée en pourcentage en

poids dans la composition considérée, dépend de la nature de ladite composition (base pour parfum ou eau de toilette par exemple) et de la puissance et de la nature de l'effet recherché au niveau produit final. Il va de soi que, dans une base pour parfum, la teneur en composé de formule **(I)** peut être très importante, par exemple supérieure à 50% en poids et peut atteindre 90% en poids, tandis que, dans un parfum, une eau de toilette ou une lotion après rasage, cette teneur pourra être très inférieure à 50% en poids.

Il peut, également. intervenir dans les shampoings parfumés à raison de 0,5 à 2% ou pour parfumer tout produit capillaire.

Ainsi, la limite inférieure de la teneur en composé de formule **(I)** peut être celle qui provoque une modification perceptible à l'odorat, de la fragrance ou de la note du produit fini. Dans certains cas, cette teneur minimale peut être de l'ordre de 0,01% en poids. On peut, évidemment. faire appel à des teneurs non comprises dans les limites des teneurs indiquées ci-avant, sans pour autant sortir du cadre de la présente invention.

Etant donné que les composés selon l'invention possèdent la double fonctionnalité tensioactive/parfumante, il peut être envisagé de prévoir, en tant qu'application desdits composés, une composition tensioactive et parfumante comprenant au moins l'un d'eux.

Il va de soi que les formulations détergentes selon l'invention décrites supra peuvent comprendre en plus ou au lieu de la composition tensioactive à base de **(I)**, de la composition parfumante à base de **(I)** et/ou de la composition tensioactive et parfumante à base de **(I)**.

Les exemples qui suivent permettront de mieux comprendre la présente invention et de faire ressortir tous ses avantages et ses variantes de mise en oeuvre.

DESCRIPTION DES FIGURES

- La **Fig. 1** est un diagramme montrant les courbes comparatives des températures de trouble mesurées en fonction du HLB pour des NOPOLS alcoxylés selon l'invention, d'une part, et pour des alcools et des nonylphénols éthoxylés, d'autre part (Exemple 3.2.a);
- La **Fig. 2** est un diagramme montrant également des courbes comparatives de températures de trouble mesurées en fonction de l'HLB pour des NOPOLS alcoxylés selon l'invention d'une part, et pour des alcools et des nonylphénols éthoxylés, d'autre part (Exemple 3.2.b);
- La **Fig. 3** est un diagramme montrant l'évolution de la température de trouble en fonction de n : courbe 1 = NOPOL n OE ; courbe 2 : NOPOL 2 OP/n OE.
- La **Fig. 4** est un diagramme montrant la variation de tension superficielle en fonction de la concentration pour du NOPOL 2 OP 5.1 OE (tension de surface statique-Exemple 3.4.1.1.);
- **La Fig. 5** est également un diagramme montrant la variation de tension superficielle en fonction de la concentration, mais cette fois pour du NOPOL 2 OP 7,5 OE (tension de surface statique à 230° C - Exemple 3.4.2.2.);
- La **Fig. 6** montre un diagramme rendant compte du pouvoir mouillant de différents NOPOLS 2 OP éthoxylés, au travers du graphe de la concentration critique dans le test NF T 73 - 406 (ISO 8022) en fonction du temps, les quatre courbes correspondant à des NOPOLS ayant des indices d'éthoxylation différents, qui sont annotés sur la figure, de même que les températures de mesure (Exemple 3.5);
- La **Fig. 7** montre un diagramme comparatif exprimant les pouvoirs détergents de NOPOL modifiés conformément à l'invention par rapport à ceux de témoin LABS et SYNPERONIC A3/A9, les courbes 1,2 et 3 correspondant aux différents supports en coton utilisés, respectivement coton CS-3, coton K10D et coton AS-2, qui sont tous trois des cotons salis standards (test détergent Exemple 4).

**EXEMPLES**

1ERE PARTIE:

OBTENTION ET PROPRIETES TENSIOACTIVES DES COMPOSES SELON L'INVENTION

EXEMPLE 1 : SYNTHESE DE NOPOL = PRECURSEUR (I').

- ESSAI 1 :
  Dans un réacteur de 100 ml, on charge, dans l'ordre, du paraformaldéhyde (1,19 g, 39,6 mmol), le toluène (10 ml) et du chlorure de zinc (0,12 g, 0,88 mmol). Le mélange est chauffé à 105° Cet le β-pinène (7,08 g, 52 mmol) est introduit en 20 minutes.
  La température est maintenue à 105° C pendant deux heures.
  Le dosage par chromatographie en phase gazeuse indique un taux de transformation du β-pinène de 51 % et une sélectivité en NOPOL de 62%.

- ESSAI 2 :
  Dans un autoclave TEFLON de 100 Ml, on charge du β-pinène (7,11 g, 52,3 mmol) et du paraformaldéhyde (1,62 g, 54 mmol). Le mélange est chauffé sous pression autogène à 180° C pendant 1 heure.
  Le dosage par chromatographie en phase gazeuse indique un taux de transformation du β-pinène de 59% et une sélectivité en NOPOL de 99%.

EXEMPLE 2 : PREPARATION DE COMPOSES DE FORMULE (Ia) DU TYPE POLYETHOXYLES ET/OU-PROPOXYLES.

Le principe de cette préparation consiste à faire réagir par condensation du 6-6 diméthylbicyclo[3.1.1]hept-2-ène-2éthanol = NOPOL avec de l'oxyde d'éthylène et/ou de propylène. Le NOPOL utilisé est celui commercialisé par la Société FLUKA. Sa pureté est supérieure à 98% et par teneur en eau est de 0,37% (Karl Fischer). Dans le présent exemple, on synthétise les cinq composés (**$I_{N2}$**) suivants (OE = oxyde d'éthyléne, OP = oxyde de propylêne).

**2.1** NOPOL 3.3 OE:

Dans un réacteur d'éthoxylation de 5 litres, on introduit du NOPOL (2 kg, 12 moles) et une solution aqueuse d'hydroxyde de potassium (50%, 7,4 g).
Le milieu réactionnel est dèshydrate à 120° C sous courant d'azote.
On chauffe ensuite à 165° C et l'oxyde d'éthylène est introduit (1,75 kg, 3,3 équivalents molaires).
Le milieu réactionnel est alors refroidi, neutralisé par l'addition d'acide acétique, jusqu'à l'obtention d'un pH de 7. Le liquide obtenu est filtré sur une terre absorbante (Clarcel DIC).
On obtient un liquide fluide de couleur jaune pâle.

**2.2** NOPOL 5.1 OE:

Dans un réacteur d'éthoxylation de 5 litres, on introduit du NOPOL (1,5 kg, 9 moles) et une solution aqueuse d'hydroxyde de potassium (50 %. 7 g). Le milieu réactionnel est déshydraté à 120° C sous courant d'azote.
On chauffe ensuite à 165° C et l'oxyde d'éthylène est introduit (2,02 kg, 5,1 équivalents molaires).Le milieu réactionnel est alors refroidi, neutralisé par l'addition d'acide acétique, jusqu'à l'obtention d'un pH de 7. Le liquide obtenu est filtré sur une terre absorbante (Clarcel DIC).
On obtient un liquide fluide de couleur jaune pâle.

**2.3** NOPOL 7,5 OE :

On procède comme pour les NOPOLS 3,3 OE et 5,1 OE.

**2.4** NOPOL 2 OP :

Dans un rèacteur d'éthoxylation de 5 litres, on introduit du NOPOL (2 kg, 12 moles) et une solution aqueuse d'hydroxyde de potassium (50%, 6,7 g).
Le milieu réactionnel est déshydraté à 120° C sous courant d'azote.
On chauffe ensuite à 170° C et l'oxyde de propylène est introduit (0,743 kg, 2 équivalents molaires).
Le milieu réactionnel est alors refroidi, neutralisé par l'addition d'acide acétique, jusqu'à l'obtention d'un pH de 7. Le liquide obtenu est filtré sur une terre absorbante (Clarcel DIC).
On obtient un liquide fluide de couleur jaune pâle.

**2.5** NOPOL 2 OP/3.3 OE :

Dans un réacteur d'éthoxylation de 5 litres, on introduit du NOPOL (1 kg, 6 moles) et une solution aqueuse d'hydroxyde de potassium (50%, 3,1 g).
Le milieu réactionnel est déshydraté à 120° C sous courant d'azote.
On chauffe ensuite à 170° C et l'oxyde de propylène est introduit (0,7 kg, 2 équivalents molaires). A la fin de l'addition de l'oxyde de propylène on introduit l'oxyde d'éthylène (0,87 kg, 5,7 équivalents molaires).
Le milieu réactionnel est alors refroidi, neutralisé par l'addition d'acide acétique, jusqu'à l'obtention d'un pH de 7. Le liquide obtenu est filtré sur une terre absorbante (Clarcel DIC).
On obtient un liquide fluide limpide de couleur jaune pâle/jaune citron, à odeur caractéristique douce, faible et agréable.

**2.6** NOPOL 2 OP/5.1 OE:

Dans un réacteur d'éthoxylation de 5 litres, on introduit du NOPOL (1 kg, 6 moles) et une solution aqueuse d'hydroxyde de potassium (50%, 3,7 g).
Le milieu réactionnel est déshydraté à 120° C sous courant d'azote.
On chauffe ensuite à 170° C et l'oxyde de propylène est introduit (0,7 kg, 2 équivalents molaires). A la fin de l'addition de l'oxyde de propylène, on introduit l'oxyde d'éthylène (1,35 kg, 5,1 équivalents molaires).
Le milieu réactionnel est alors refroidi, neutralisé par l'addition d'acide acétique, jusqu'à l'obtention d'un pH de 7. Le liquide obtenu est filtré sur une terre absorbante (Clarcel DIC).
On obtient un liquide fluide limpide de couleur jaune pâle/jaune citron, à odeur caractéristique.

**2.7** NOPOLS 2 OP/7,5 OE et 2 OP/10,3 OE :

On procède comme pour les NOPOLS 2 OP/3,3 OE et 2 OP/5,1 OE.
Le NOPOL 2 OP/7,5 OE se présente sous forme de liquide jaune citron à odeur caractéristique.
Le NOPOL 2 OP/10,3 OE obtenu est un solide crémeux à 20° C, liquide vers 25°C opaque, de couleur jaune clair à odeur caractéristique.
Les produits mixtes OP/OE 2,5 à 2,7 contiennent une proportion importante de NOPOL 2 OP libre et une quantité non négligeable de polypropylène glycol (PPG) ou de copolymère polypropylène-polyéthylène glycol (PPG-PEG).
Le Tableau ci-dessous rend compte de cela

| ECHANTILLONS (proportions : % en masse) | NOPOL 2 OP 3,3 OE | NOPOL 2 OP 5.1 OE | NOPOL 2 OP 7,5 OE | NOPOL 2 OP 10.3 OE |
|---|---|---|---|---|
| NOPOL 2 OP libre | 49 | 32 | 17 | 11 |
| PPG-PEG ou PPG libre | 12.5 | 12 | 4,7 | 9.5 |

Il est possible qu'une fraction importante d'espèces moléculaires libres, surtout pour les échantillons à 3,3 et 5,1 OE (respectivement environ 60 et 40%), ait une influence sur les propriétés physico-chimiques étudiées. La présence d'une forte proportion de NOPOL 2 OP peut être utile.
Les performances des tensioactifs éthoxylés dépendent dans une large mesure du "profil" de répartition des maillons d'oxyde d'éthylène comme de la proportion des molécules non éthoxylées.

**2.8** ANALYSE CHIMIQUE ;

L'analyse chimique (indice d'hydroxyle et indice de base sur les condensats bruts de réaction) a conduit aux résultats suivants :

| | IOH trouvé | IOH théorique | IB |
|---|---|---|---|
| NOPOL | 317,8 | 337,5 | - |
| NOPOL 3.3 OE | 180,9 | 177,2 | 0,90 |
| NOPOL 5.1 OE | 145,0 | 143,4 | 0,70 |
| NOPOL 7.5 OE | 113,0 | 113,0 | 1,60 |
| NOPOL 2 OP | 212,8 | 198,7 | 1,01 |
| NOPOL 2 OP/3.3 OE | 143,5 | 130,8 | 0,60 |
| NOPOL 2 OP/5.1 OE | 117,8 | 110,8 | 0,55 |
| NOPOL 2 OP/7.5 OE | 90,2 | 91,1 | 1,15 |

- Indice de base IB :
obtenu par potentiométrie et exprimé en milligrammes d'hydroxyde de potassium par gramme de produit brut.
- Indice d'hydroxyle IOH :
obtenu par la méthode d'acétylation comme typiquement décrite dans R. STETZLER Anal. Chem., 34, 194 (1962).

EXEMPLE 3 : PROPRIETES DES NOPOLS OE ET/OU OP.

**3.1** TEMPERATURES DE TROUBLE :

Cette mesure permet d'apprécier les propriétés tensioactives des NOPOLS préparés comme indiqué ci-avant dans l'exemple 1.

| n | POINT DE TROUBLE (° C) | | | |
|---|---|---|---|---|
| | BDG | | 0,5 % ED | |
| | NOPOL 2 OP + n OE | NOPOL n OE | NOPOL 2 OP + n OE | NOPOL n OE |
| 3,3 | 55,7 | 56,9 | - | - |
| 5,1 | 65,5 | 70,2 | < 5 | < 5 |
| 7,5 | 74,9 | 80,6 | 53 | 66,8 |
| 10,3 | 81,0 | - | 77 | - |

- BDG = butyldiglycol,
- ED = eau distillée.

**3.2** COMPARAISON AVEC LES ALCOOLS ET LES NONYLPHENOLS ETHOXYLES :

a) NOPOL 3,3 OE (HLB 9,4) et NOPOL 5,1 OE (HLB 11,5) :

Ces deux produits sont comparés à :

- la gamme des alcools en $C_{12}/C_{14}$éthoxylés (LA 20-LA 30-LA 40),
- la gamme des nonylphénols éthoxylés (NP3-NP4-NP5-NP6 et NP7).

A HLB égal, les NOPOLS éthoxylés 3,3 et 5,1 ont des températures de trouble nettement inférieures aux alcools éthoxylés et sensiblement égales à celles des nonylphénols éthoxylés.
La **fig. 1** annexée montre les courbes des températures de trouble mesurées en fonction du HLB. Les mesures sont réalisées à des concentrations de 10 % dans le BDG à 25% dans l'eau.

b) NOPOL 7,5 OE (HLB 13,3) :

Même constatation que ci-dessus en comparant ce produit avec les :

- nonylphénols éthoxylés (NP8-NP9-NP10-NP1064 et NP12),
- l'alcool éthoxylé : $C_{12}/C_{14}$ - 9 OE (LA 90),
- l'alcool èthoxylé : $C_{10}/C_{12}$- 5,2 OE (DB 311)

La **fig. 2** annexée montre les courbes des températures de trouble mesurées en fonction de l'HLB. Les mesures sont effectuées à des concentrations de 5 g/litre d'eau distillée.

**c)** NOPOLS n OE et, 2 OP/n OE :

L'évolution de la température de trouble en fonction de n est donnée par la **fig. 3** annexée :

- courbe 1 = NOPOL n OE
- courbe 2 = NOPOL 2 OP/n OE.

Les mesures sont réalisées à des concentrations de 10 % dans le BDG à 25 % dans l'eau.

**3.3** MESURES DE pH

pH à 1 % dans l'eau distillée

| | |
|---|---|
| - nopol 2 OP 3,3 OE : | environ 6,5 |
| - nopol 2 OP 5,1 OE : | environ 6,4 |
| - nopol 2 OP 7,5 OE : | environ 6,0 |
| - nopol 2 OP 10,3 OE : | environ 6,1 |

**3.4** CARACTERISTIQUES DE SURFACE

Seuls sont étudiés les nopols 2 OP / 5.1 et 7.5 OE.

***3.4.1*** *Tension superficielle statique et CMC*

**3.4.1.1** Nopol 2 OP 5.1 OE

Les dilutions successives sont effectuées en fioles jaugées de 200 ml à l'eau distillée à partir de solutions mères à 50 g/l et 20 g/l.

La tension superficielle à chaque concentration est effectuée à température ambiante (= 23° C) à l'aide d'un tensiomètre PROLABO "Tensimat n° 3" muni d'une lame de platine.

**Resultats**

| | |
|---|---|
| - $\gamma_S$ à 0,001 g/l = | 69,1 mN/m |
| - $\gamma_S$à 0,01 g/l = | 60,0 mN/m |
| - $\gamma_S$à 0,1 g/l = | 48,6 mN/m |
| - $\gamma_S$ à 0,5 g/l = | 37,0 mN/m |
| **- $\gamma_S$ à 1 g/l =** | **32,6 mN/m** |
| - $\gamma_S$ à 2g/l = | 31,6 mN/m |

- $\gamma_S$ à 5 g/l = 31,9 mN/m
- $\gamma_S$ à 10g/l = 31,8 mN/m
- $\gamma_S$ à 50g/l = 32,0 mN/m

**La Fig. 4 annexée** donne la variation de tension superficielle en fonction de la concentration. La concentration micellaire critique (CMC) correspond à la rupture de pente.

- CMC du nopol 2 OP 5,1 OE : 1,0 + 0,3 g/l

La CMC de ce tensioactif correspond pratiquement au début de la zone de trouble. (Rappel : CMC du NOPOL 5,1 OE : 2,0 ± 0,5 g/l).

**3.4.2.2** Nopol 2 OP 7.5 OE

Comme précédemment, les dilutions successives sont effectuées en fioles jaugées de 200 ml à l'eau distillée à partir de solutions mères à 50 g/l et 20 g/l.

La majeure partie des mesures sont effectuées avec un appareil LAUDA TVT1, prêté par la société PROLABO ,utilisant le principe de la goutte pendante :

La tension superficielle d'un liquide est déduite de la mesure du volume des gouttes formées à l'extrémité d'un capillaire de rayon défini La force superficielle exercée sur le périmètre du capillaire étant équilibrée par le poids de la goutte pendante.

Ce tensiomètre permet la détermination de tensions superficielles et interfaciales statiques ou dynamiques entre 0,5 et 100 mM/m avec une reproductibilité donnée comme meilleure que 0,03 mM/m et une précision absolue de 1 % sur toute la gamme.

(Mesures à 23° C).

**Resultats**

- $\gamma_S$ à 0,001 g/l = 70,2 mN/m
- $\gamma_S$ à 0,01 g/l = 59,6 mN/m
- $\gamma_S$ à 0.1 g/l = 49,5 mN/m
- $\gamma_S$ à 0,5 g/l = 40,4 mN/m
- **$\gamma_S$ à 1,0 g/l = 35,1 mN/m**
- $\gamma_S$ à 5,0 g/l = 32,5 mN/m
- $\gamma_S$ à 10 g/l = 33,1 mN/m

Quelques points complémentaires sont mesurés (à 25° C) avec le tensimat n° 3 selon la méthode classique de la lame de platine :

- $\gamma_S$ à 2g/l = 31,4 mN/m
- $\gamma_S$ à 3g/l = 31,4 mN/m
- $\gamma_S$ à 50 g/l = 32,7 mN/m

**La Fig. 5 annexée** montre que les mesures effectuées à la lame de platine s'intègrent bien à celles obtenues avec l'appareil à goutte pendante.

- CMC du nopol 2 OP 7,5 OE : 2 ± 0,5 g/l

(Rappel : CMC du nopol 7,5 OE 40 ± 0,5 g/l).

**Conclusion**

LA CMC des nopols 2 OP éthoxylés est encore élevée malgré un gain sensible par rapport aux nopols non oxypropylés (concentration micellaire critique divisée par deux).

**3.4.2** *Tension superficielle dynamique*

Les mesures sont faites à 1 g/l, à la température de 23 ± 0,5 °C avec une fréquence de bullage de 1s-1 sur un appareil SENSADYNE 6000.

**Résultats**

| TENSIOACTIF | $\gamma_D$ (mM/m) à 1 gramme/litre |
|---|---|
| NOPOL 2 OP 3,3 OE | 35,0 |
| NOPOL 2 OP 5,1 OE | 36,6 |
| NOPOL 2 OP 7,5 OE | 38,7 |
| NOPOL 2 OP 10,3 OE | 42,9 |

Les nopols 2 OP éthoxylés ont une tension dynamique basse, très voisine de leur tension statique

(nopol 2 OP 5,1 OE : $\gamma_S$ = 32,6 mN/m)
(nopol 2 OP 7,5 OE : $\gamma_S$ = 35,1 mN/m)

**3.5** POUVOIR MOUILLANT

Selon la norme NF T 73 - 406 (ISO 8022) "Mesure du pouvoir mouillant". Les solutions sont préparées dans l'eau distillée et les mesures effectuées à 20 ± 1 °C (Cf **Fig. 6 annexée)**.
Le pouvoir mouillant est exprimé par la concentration telle qu'un disque de coton écru, placé au sein de la solution, commence à s'enfoncer au bout de 100 secondes.

- NOPOL 2 OP 5,1 OE
  Les solutions testées commencent à présenter une certaine opalescence à le concentration correspondant au pouvoir mouillant A 0,5 g/l et au dessous. elles sont parfaitement limpides.
  Le pouvoir mouillant du nopol 2 OP 5,1 OE est également mesuré à 50 °C pour évaluer l'écart d'efficacité entre cette température et l'ambiante.

  pouvoir mouillant à 20 ° C = **0,86 ± 0,02 g/l**
  ou 63,6 secondes pour 1 g/l.
  pouvoir mouillant à 50 °C = **0,58 ± 0,01 g/l**
  ou 37,3 secondes pour 1 g/l.

- NOPOL 2 OP 7,5 OE

  pouvoir mouillant à 20° C = **1,20 ± 0,02 g/l**
  ou 179,5 secondes pour 1 g/l.

- NOPOL 2 OP 10,3 OE

  pouvoir mouillant à 20° C = **1,85 ± 0,03 g/l**
  ou environ 600 secondes pour 1 g/l.

Les mesures sont faites à une concentration proche de la CMC. La pente des droites exprimant le mouillage est très faible. Autrement dit le temps de mouillage varie très vite avec la concentration de tensioactif (voir **Fig. 5** annexée). A 50° C le pouvoir mouillant est améliore.

2EME PARTIE : DETERGENCE

Les produits terpéniques de l'invention peuvent, en outre, être utilisés comme agents de nettoyage industriel de surfaces métalliques.
La méthode de mesure du pouvoir détergent des produits terpéniques de l'invention a été réalisée comme suit

POUVOIR DETERGENT :

- *PRINCIPE :*
Le test simule un lavage en machine simplifié, à l'aide d'un tergotomètre. Il consiste à laver à 40 °C des éprouvettes de tissus salis de façon standard et uniforme, avec seulement le produit à tester et du bicarbonate de sodium pour stabiliser le pH. Le lavage dure trente minutes et la détergence est évaluée par mesure de la blancheur des pièces de tissu, avant et après lavage, à l'aide d'un colorimètre.
- *APPAREILLAGE ET MATERIEL :*

. MATERIEL :

* Tergotomètre : "U.S. TESTING Co Inc" HOBOKEN N.J. Modèle 7243,
* Glaceuse pour repasser les échantillons de tissu après lavage,
* Appareil de mesure de couleurs "Dr Lange LUCI 100" .

. TISSUS :
Les tissus standards sont fabriqués par le CFT (Center For Test materials) ou par la société "Test Fabric". Ils ont les caractéristiques suivantes.

| Tissus salis standards | (salissure) | nombre de pièces par essai |
|---|---|---|
| - Coton sali CS-3 CFT | (vin) | 2 |
| - Coton sali K 10D CFT | (huile minérale et noir de carbone) | 2 |
| - Coton sali AS-2 CFT | (huile minérale et noir de carbone) | 2 |
| - Polyester coton E MPA 104 CFT | (huile minérale et noir de carbone) | 2 |
| - Polyester Dacron type 54 Test Fabric | (huile minérale et noir de carbone) | 2 |
| Tissus blancs standards | | |
| - Coton CN 1 blanc, non sali, du CFT | | 5 |
| - Polyester coton PCN 1, blanc, non sali, du CFT | | 5 |

soit, pour chaque essai, un total de 20 pièces de tissus.

. PRODUITS :
Le "LABS", dodécylbenzènesulfonate de sodium technique (ALDRICH) est le produit témoin pris pour référence.

- *MODE OPERATOIRE :*

. TEST DE DETERGENCE :
Le tergotomètre est un appareil constitué de 4 pots de 2 litres en inox sur lesquels sont adaptés des agitateurs que l'on règle à 50 cycles par minute (100 allers-retours). Les pots sont placés dans une cuve d'eau régulée à 40 °C.
Dans chaque pot on met 1 Titre d'eau permutée contenant :

* 1 gramme de tensioactif
* 1 gramme de bicarbonate de soude.

Quand l'eau est en température, les pots sont introduits dans le bain thermostaté en déclenchant simultanément l'agitation et un chronomètre.
En fin de lavage (30 minutes) les bains sont récupérés (250 cc) pour contrôle du pH.
Les tissus sont rincés trois fois à l'eau de ville, puis essorés à la main et séchés à plat individuellement entre deux feuilles de papier blanc absorbant.
Les tissus sont à nouveau mis entre deux feuilles de papier absorbant propres, et repassés dans la glaceuse à une température voisine de 110° C.

. MESURE DE COULEURS :
Les mesures sont effectuées avec le colorimètre "LUCI 100" avant et après lavage selon le système "L", "a", "b" (Echelle du noir au blanc, du vert au rouge, et du bleu au jaune) pour mesurer le pouvoir détergent des tensioactifs essayés (augmentation de la blancheur des pièces de tissus salis).

. CALCUL DE LA DETERGENCE :
La valeur "DE" (détergence) est calculée pour chaque type de tissu en faisant la somme géométrique des écarts de couleur DL, Da et Db avant et après lavage sur les tissus salis.
Soit détergence $DE = (DL^2+Da^2+Db^2)^{1/2}$.

EXEMPLE 4.

| OP (p) | OE (q) | |
|---|---|---|
| 2 | 3,3 | produit 1 |
| 2 | 5,1 | produit 2 |
| 2 | 7,5 | produit 3 |
| 2 | 10,3 | produit 4 |

Pouvoir détergent mesuré selon le test de détergence décrit ci-dessus (lavage simulé à l'aide d'un tergotomètre) : la détergence cumulée, mesurée sur les différents types de tissus, aboutit aux résultats suivants, en prenant le LABS (dodécylbenzènesulfonate de ALDRICH) pour référence avec une détergence "DE" égale à 100.

| | "DE" |
|---|---|
| .LABS | 100 |
| . mélange classique de tensio-actifs non-ioniques (synperonic A3/A9) pour les compositions détergentes, constitué d'alcools gras en C13 éthoxylé à 3 OE + alcools gras en C13 éthoxylé à 9 OE selon un rapport 30/70 en poids | 96 |
| . produit 2 | 89 |
| . mélange des produits 1+2+3+4 selon un rapport 10/50/20/20 | 94 |

L'action détergente est globalement la même sur les tissus de cotons salis (CS3, K10D, AS2) avec tous les tensioactifs étudiés (Cf. **Fig. 7 annexée**).

EXEMPLE 5 : UTILISATION DU MELANGE PRODUITS 1 + 2 + 3 + 4 SELON FORMULATION DETERGENTE COMPACTE POUR LE LINGE.

| | |
|---|---|
| - Alkylbenzène sulfonate de sodium linéaire : | 3 % |
| - Produits 1+2+3+4 (10/50/20/20) : | 8 % |
| - NABION 15® (cogranulé carbonate silicate commercialisé par RHONE-POULENC) | 40 % |
| - Perborate tétrahydrate | 16 % |
| - CarboxyMethylcellulose sodique | 0,6 % |
| - Enzymes (Savinase/alcalase) | 0,6 % |
| - Azurants optiques en mélange | 0,14% |
| - TétraAcétylEthylèneDiamine (activateur du perborate) | 4,3 % |
| - Sel sodique de l'hydroxyethylène 1-1 diphosphonate | 1,6 % |
| - SOKALAN CP5® (copolymère acrylique/maleique commercialisé par BASF) | 5 % |
| - Carbonate de sodium léger absorbant | 6 % |
| - Antimousse Silicone solide à 8 % en silicone | 2,5 % |
| - Parfum | 0,12% |
| - Sulfate de sodium | pour compléter à 100 % |

EXEMPLE 6 : UTILISATION DU MELANGE PRODUITS 1 + 2 + 3 + 4 SELON FORMULATION DETERGENTE POUR LE LINGE.

| | |
|---|---|
| - Alkylbenzène sulfonate de sodium linéaire | 7,5 % |
| - Produits 1+2+3+4 (10/50/20/20) | 4,5 % |
| - Zéolithe TEL | 24 % |
| - Disilicate ALNA | 2,5 % |
| - Perborate tétrahydrate | 14 % |
| - CarboxyMethylcellulose sodique | 0,6 % |
| - Enzymes (Estérase) | 0,3 % |
| - Azurants optiques en mélange | 0,11 % |
| - TétraAcétylEthylèneDiamine (activateur du perborate) | 2 % |
| - Sel sodique de l'hydroxyethylène 1-1 diphosphonate | 1,6 % |
| - SOKALAN CP5® (copolymère acrylique/maleique commercialisé par BASF) | 4 % |

| | |
|---|---|
| - Carbonate de sodium | 14 % |
| - Antimousse | 1,5 % |
| - Repell-O-Tex® (agent anti-salissure commercialisé par RHONE-POULENC) | 0,1 % |
| - Parfum | 0,1 % |
| - Sulfate de sodium | pour compléter à 100 % |

EXEMPLE 7 : ESSAIS DETERGENCE EN FORMULATION LESSIVIELLE COMPLETE

Le test mis en oeuvre est celui décrit en préambule à l'exemple 4.
Pour ces essais. on a remplacé le mélange de NOPOLS 1 + 2 + 3 + 4 par des alcools gras éthoxylés du type synperonic $A_5$, $A_7$ et $A_9$ et par du LABS. ces produits constituants des témoins, ainsi que par du NOPOL produit 2 selon l'exemple 4.
En prenant le LABS comme référence avec une base = 100, les résultats en DE cumulée trouvés sont les suivants :

| | |
|---|---|
| Synperonic $A_5$ | 110 |
| Synperonic $A_7$ | 121 |
| Synperonic $A_9$ | 113 |
| NOPOL mélange OE | 104 |
| **NOPOL 5,1 OE** | **110** |

EXEMPLE 8 : FORMULE DEGRAISSANTE DE TOLES METALLIQUES S, A BASE DE NOPOL A 5,1 OE.

| | | |
|---|---|---|
| - NOPOL 5,1 OE | | 5 % |
| - MIRANOL J2M Conc. (di sodium caprylamphodiacétate) | | 11,7 % |
| - triéthanolamine | | 5 % |
| - butyldiglycol | | 5 % |
| - NERVANAID NTA Na3 (nitrilotriacétate de sodium commercialisé par Rhône-Poulenc) | | 10 % |
| - parfum et colorant | | |
| - eau | complément à | 100 % |

Cette formulation est diluée de 10 à 100 fois.

EXEMPLE 9 : DEGRAISSAGE DES TOLES METALLIQUES A L'AIDE DE FORMULATIONS COMPRENANT LES COMPOSES (**Ia**) SELON L'INVENTION : TEST DE DETERGENCE.

**9.1** MODE OPERATOIRE :

Le test comporte trois étapes :

**1)** *GRAISSAGE* de plaques en acier prédégraissées de marque "Q-Panel". Stock n° R-36 type "Dull matt finish" de 0,8 x 76 x 152 mm.
Les plaques sont immergées 2 minutes dans les huiles à tester, puis suspendues pour égouttage pendant 24 heures.
**2)** *NETTOYAGE:*
Les plaques graissées sont immergées dans la solution détergente à 50 °C, constituée par dilution à 20 g/l d'une lessive basique "PARCO 8805" (PARKER) et contenant 1 g/l (matière active) du tensioactif à tester. Les essais sont faits dans l'eau permutee.
**3)** *RINNÇAGE ET NOTATION :*
Les plaques sont rincées sous un filet d'eau courante 5 secondes sur chaque face (débit = 2 l/min ; température entre 15 et 17 °C). Elles sont notées de 0 à 4 suivant la proportion de surface recouverte par un film d'eau continu, c'est-à-dire la proportion de la surface exempte de toute trace d'huile résiduelle.
"0" correspond à une absence totale de moiullage.
"4" correspond à un mouillage parfait de toute la plaque sur les deux faces (voir annexes).
Le temps d'immersion de la plaque dans la solution détergente est choisi pour permettre un dégraissage complet (notation 4) avec le produit de référence (IGEPAL NP 10) et une absence de dégraissage (notation 0) avec la solution détergente ne contenant que la lessive diluée de base, sans tensioactif additionnel. Les huiles utilisées sont :

- une huile paraffinique TOTAL® 200 Neutral. Le temps d'immersion de la plaque dans la solution détergente est de 1 minute,
- un triglycéride, l'huile de colza PHYTOROB® PHT, difficile à éliminer. La procédure suivie comporte cinq étapes de lavage et rinçage, pour une durée totale d'immersion de 15 minutes. Cette durée d'immersion est nécessaire (théoriquement) pour obtenir un nettoyage complet de la plaque avec IGEPAL® NP 10 de référence.

**9.2** TENSIOACTIFS TESTES :

- Lessive PARCO® 8805 de PARKER seule (sans tensioactif) [solution concentrée (à 45 % d'extrait sec) et diluée à 20 g/l],
- IGEPAL® NP 10 [Nonylphénol à 10 oxydes d'éthylène],

- PLURAFAC® LF 431 de BASF® [Alcool OE/OP bloqué $CH_3$],
- NOPOL 7,5 OE + 5,5 OP [composé Ia],
- NOPOL 7,5 OE [composé Ia],
- NOPOL 2 OP 3,3 OE [composé Ia],
- NOPOL 2 OP 5,1 OE [composé Ia],
- NOPOL 2 OP 7,5 OE [composé Ia],
- NOPOL 2 OP 10,3 OE [composé Ia].

Les composés (Ia) sont préparés comme décrit dans les exemples précédents.

**9.3** DETERGENCE VIS-A-VIS DE L'HUILE PARAFFINQUE :

Le temps d'immersion de la plaque dans la solution détergente est de 1 minute.

TABLEAU 1

| DEGRAISSAGE METALLURGIQUE A 50 °C | | | |
|---|---|---|---|
| HUILE PARAFFINIQUE 200 NEUTRAL® | | | |
| Lessive (20 g/l) + tensioactif (1 g/l) | 1 min notation | Lessive (20 g/l) + tensioactif (1 g/l) | 1 min notation |
| Lessive PARCO 8805 seule | 0 | NOPOL 2 OP 5,1 OE | 3 + |
| IGEPAL NP 10 | 4 | NOPOL 2 OP 7,5 OE | 4 |
| NOPOL 7,5 OE | 4 | NOPOL 2 OP 10,3 OE | 4 |
| NOPOL 7,5 OE + 5,5 OP | 4 | PLURAFAC LF 431 | 4 |
| NOPOL 2 OP 3,3 OE | 3 | - | - |

La lessive seule ne permet pas de dégraisser les plaques recouvertes d'huile 200 NEUTRAL.
Par contre, le résultat est excellent avec 1 g/l d'IGEPAL NP 10 et de PLURAFAC LF 431.
Les nopols 7,5 OE et 7,5 OE + 5,5 OP sont également excellents.
Dans la série des nopols 2 OP, il est préférable d'avoir au moins 7,5 oxydes d'éthylène pour avoir un dégraissage partait après 1 minute.

**9.4** DETERGENCE VIS-A-VIS DE L'HUILE COLZA :

Le temps d'immersion dans la solution détergente est de 15 minutes.
La lessive seule ne donne aucun résultat au bout de ce temps. Par contre, l'IGEPAL NP 10 dégraisse totalement la plaque Q-Panel après 10 minutes d'immersion.

TABLEAU 2

| Degraissage Metallurgique a 50 °C | | | | | |
|---|---|---|---|---|---|
| Huile de Colza | | | | | |
| Lessive (20 g/l) + Tensioactif (1 g/l) | Notation | | | | |
| | 1 min | 2 min | 5 min | 10 min | 15 min |
| Lessive PARCO 8805 seule | 0 | 0 | 0 | 0 | 0 |
| IGEPAL NP 10 | 0 | 1 | 2 | 4 | |
| NOPOL 7.5 OE + 5.5 OP | 0 | 0 | 1 | 1 | 2 |
| NOPOL 2 OP 3.3 OE | 0 | 0 | 0 | 0 | 0 |
| NOPOL 2 OP 5.1 OE | 0 | 0 | 1 | 1 + | 2 + |
| NOPOL 2 OP 7.5 OE | 0 | 1 | 2 | 2 + | 3 |
| NOPOL 2 OP 10.3 OE | 0 | 1 | 1 + | 1 + | 2 |
| PLURAFAC LF 431 | 0 | 1 | 1 + | 1 + | 2 |

Parmi les nopols éthoxylés, le meilleur produit est le nopol 2 OP 7,5 OE qui atteint la note 3.
Le produit PLURAFAC LF 43 I n'est pas le meilleur détergent de l'huile de colza.

EXEMPLE 10 : EVALUATION DE DERIVES DE NOPOL (Ia) - TEST DE MOUSSAGE.

**10.1** CONDITIONS OPERATOIRES :

Le test utilisé est le suivant.
La solution de tensioactif (masse = 900 g) est agitée au moyen d'une paie centripète à 2 000 tours/min pendant 5 min.
Le diamètre de la turbine d'agitation est de 40 mm.
L'évolution du volume de mousse est tracée en fonction du temps. Les températures de trouble des solutions basiques sont déterminées.
Les dérivés du nopol testés sont : NOPOL 2 OP + 5,1 OE et NOPOL 7,5 OE + 5,50 OP.
En milieu basique, le NOPOL 2 OP + 5, 1 OE, ainsi que le NOPOL 7,5 OE + 5,5 OP ne sont absolument pas moussants à 20 °C comme à 50 °C.
Les produits utilisés sont :

- ANTAROX® BL 330 (alcool éthoxylé bloqué chloré),
- IGEPAL® NP 1064 (nôylphénol éthoxylé à 10 OE),
- IGEPAL® NP 9 (nonylphénol éthoxylé à 9 OE),
- PLURAFAC® LF 431 (BASF) (phosphate ester),
- TETRONIC 701,
- SAPONINE (PROLABO).

Les nopols mis en oeuvre ont un pouvoir dètergent au moins aussi bon que les témoins.

**10.2** POLVOIR MOUSSANT EN MILIEU BASIQUE A 20 °C ET A 50 °C :

CONDITIONS DU TEST :

- 1 g/l de tensioactif testé,
- 20 g/l de lessive basique PARCO® 8801.

RESULTATS : cf. tableau 3 ci-après.

Dans les conditions de ce test, le NOPOL 2 OP + 5,1 OE et le NOPOL 5,5 OP + 7,5 OE sont des tensioactifs non moussants, tant à 20 °C qu'à 50 °C. Leurs performances se rapprochent de celles du TETRONIC® 701 (PUCK), sachant que les composés (Ia) sont de meilleurs détergents que ces TETRONIC®.

**10.3** TEMERATURES DE TROUBLE :

- IGEPAL® NP 1064 61 °C
- ANTAROX® BL 330 22 °C
- TETRONIC® 701 22 °C
- NOPOL 7,5 OE + 5,5 OP < 20 °C
- NOPOL 2 OP + 5,1 OE 6 °C

**TABLEAU 3**

| VOLUME DE MOUSSE (ML) | 0 min | 1 min | 2 min | 3 min | 4 min | 5 min | 10 min | 15 min |
|---|---|---|---|---|---|---|---|---|
| 20 °C | | | | | | | | |
| Bon moussant IGEPAL 1064 | | 1170 | 1070 | 1010 | 960 | 940 | 850 | 700 |
| Peu moussant ANTAROX BL 330 | 120 | 80 | 60 | 50 | 50 | 50 | 30 | 20 |
| Pas moussant TETRONIC 701 | 0 | 0 | 0 | 0 | 0 | 0 | 0 | 0 |
| NOPOL 7,5 OE + 5,5 OP | 0 | 0 | 0 | 0 | 0 | 0 | 0 | 0 |
| NOPOL 2 OP + 5,1 OE | 5 | 5 | 5 | 5 | 5 | 5 | 0 | 0 |
| 50 °C | | | | | | | | |
| Bon moussant IGEPAL NP 1064 | 860 | 700 | 600 | 550 | 530 | 490 | 420 | 330 |
| Peu moussant IGEPAL NP 9 | 100 | 70 | 50 | 50 | 30 | 30 | 30 | |
| Très peu moussant ANTAROX BL 330 | 30 | 10 | 0 | 0 | 0 | 0 | 0 | 0 |
| Pas moussant TETRONIC 701 | 0 | 0 | 0 | 0 | 0 | 0 | 0 | 0 |
| NOPOL 7,5 OE + 5,5 OP | 0 | 0 | 0 | 0 | 0 | 0 | 0 | 0 |
| NOPOL 2 OP + 5,1 OE | 0 | 0 | 0 | 0 | 0 | 0 | 0 | 0 |

## Revendications

1. Composés de formule suivante :

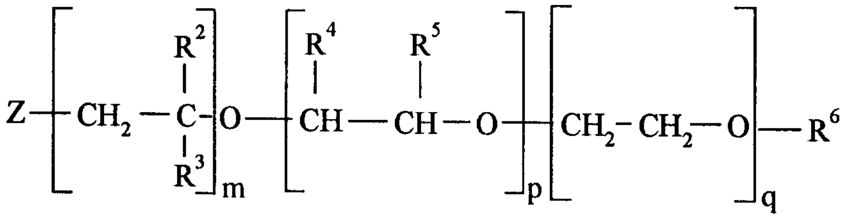

(I)

dans laquelle :

* Z représente un radical bicyclo[a,b,c]heptényle ou bicyclo[a,b,c]heptyle, avec :

⊕ a + b + c = 5,
⊕ a = 2, 3 ou 4,
⊕ b = 2 ou 1,
⊕ c = 0, 1,

ledit radical étant éventuellement substitué par au moins un alkyle de $C_1$-$C_6$, de préférence un méthyle et comprenant un squelette choisi parmi ceux mentionnés ci-après (**Z**$_1$ à **Z**$_7$), ainsi que parmi leurs correspondants heptyle sans double liaison :

1) 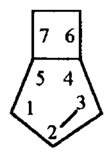 [3.2.0]

2) 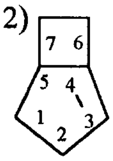 [3.2.0]

3) 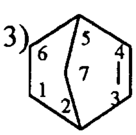 [2.2.1]

4) 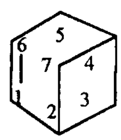 [3.1.1]

5) 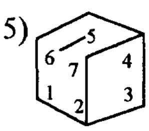 [3.1.1]

6) 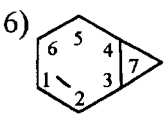 [4.1.0]

7) 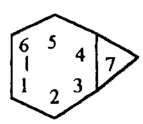 [4.1.0]

▷ $R^2$ et $R^3$ sont identiques ou différents et représentent l'hydrogène ou un groupement (cyclo)alkyle ou (cyclo)alcényle, linéaire ou ramifié, de préférence (cyclo)alkyle linéaire ou ramifié en $C_1$-$C_{22}$, l'hydrogène et le méthyle étant plus particulièrement préférés;

▷ $R^4$ et $R^5$ sont identiques ou différents et représentant l'hydrogène ou un radical (cyclo)alkyl ou (cyclo)alcényle linéaire ou ramifié, de préférence (cyclo) alkyle linéaire ou ramifié en $C_1$-$C_{22}$, l'hydrogène, le méthyle et l'éthyle étant plus particulièrement préférés,

▷ $R^6$ correspond à l'hydrogène ou à l'un des groupements suivants : $SO_3M$, $OPO_3(M)_2$, $-(CH_2)_m$--COOM avec m compris entre 1 et 6, $-(CH_2)_z$--$SO_3M$ avec z = 1 à 6 et dans lesquels M = H, Na, K, Li ou $N(R^7)_4^+$ avec $R^7$ = H ou un (cyclo)alkyle en $C_1$-$C_{22}$, de préférence $C_1$-$C_6$, éventuellement hydroxylé, l'hydrogène le méthyle et l'hydroxyéthyle étant préférés.

▷

. $m = 0, 1$
. $1 \leq p \leq 20$, de préférence $1 \leq p \leq 10$, et plus préférentiellement encore $1 \leq p \leq 3$.
. $1 \leq q \leq 200$, de préférence $1 \leq q \leq 50$ et, plus préférentiellement encore, $1 \leq q \leq 10$.

▷ avec la condition selon laquelle quand m = 0, au moins l'un des substituants $R^4$, $R^5$ est différent de l'hydrogène.

**2.** Composés selon la revendication 1, caractérisés en ce que leur radical Z est substitué sur au moins l'un de ses carbones par au moins deux groupements alkyles en $C_1$-$C_6$, de préférence deux méthyles en $C_7$.

**3.** Composés selon la revendication 1 ou 2, caractérisés en ce que m = 1 (composés **Ia**) et en ce que leur radical Z est sélectionné dans la liste comportant les radicaux $Z_3$ à $Z_7$, tels que définis dans la revendication 1.

**4.** Composés selon la revendication 3, caractérisés en ce que Z = $Z_4$ ou $Z_5$

**5.** Composés selon la revendication 1 ou 2, caractérisés en ce que m = 0 (composés **Ib**) et en ce que le radical Z comprend le correspondant heptyle sans double liaison du squelette Z3.

**6.** Composés selon la revendication 5, caractérisés en ce que le squelette de la formule **(I)** est substitué par au moins un reste alkyle - avantageusement en $C_1$-$C_6$ - de préférence par au moins un méthyle sur le carbone 2 ou 5 du bicycle.

**7.** Composés selon l'une quelconque des revendications 1 à 6, caractérisés en ce que les motifs récurrents de la formule **(I)**, identifiés par les paramètres p et q, sont présents en quantité telle et sont agencés de telle sorte qu'ils forment une chaîne polymère statistique ou séquencée, de préférence séquencée dans l'ordre donné par la Formule **(I)**.

**8.** Procédé de préparation des composés selon l'une quelconque des revendications 1 à 4 et 7, et pour lesquels m = 1 dans la formule **(I)**; caractérisé :

- en ce qu'il consiste essentiellement à faire réagir un réactif de formule **(I')** :

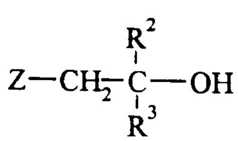

**(I')**

avec un réactif **(Wop)** de formule :

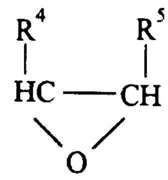

**(Wop)**

et, de préférence puis,
avec un réactif **(Woe)** de formule :

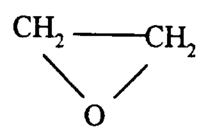

Formules dans lesquelles

- ▷ Z est tel que défini dans les revendications 1, 2, 3 ou 4,
- ▷ $R^2$ et $R^3$ sont identiques ou différents et représentent l'hydrogène, un (cyclo)alkyle, un (cyclo)alcényle linéaire ou ramifié en $C_1$-$C_{22}$, l'hydrogène et le méthyle étant plus particulièrement préférés.
- ▷ $R^4$ et $R^5$ sont identiques ou différents et représentent l'hydrogène ou un radical (cyclo)alkyl ou (cyclo)alcényle linéaire ou ramifié, de préférence (cyclo)alkyle linéaire ou ramifié en $C_1$-$C_{22}$, l'hydrogène, le méthyle et l'éthyle étant plus particulièrement préférés, avec la condition selon laquelle au moins l'un des substituants $R^4$, $R^5$ est différent de l'hydrogène;

pour obtenir un produit **(I.1)** :

**(I.1)**

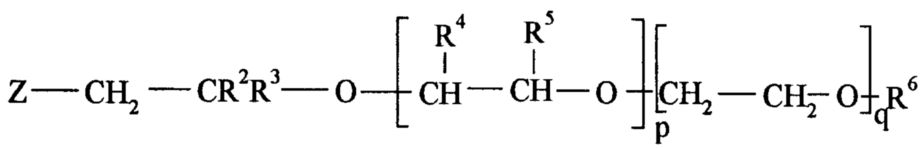

avec p, q et $R^6$ sont tels que définis dans la revendication 1.

- et en ce que l'on effectue cette réaction en présence d'une quantité efficace de catalyseur et à une température supérieure à 100° C, de préférence comprise entre 120° et 250° C et, plus préférentiellement encore, comprise entre 150° C et 200° C.

**9.** Procédé de préparation des composés selon l'une quelconque des revendications 1, 2, 5, 6 ou 7 pour lesquels m = 0 dans la formule **(I)**,
caractérisé :

- en ce qu'il consiste essentiellement à faire réagir un réactif de formule

(I'') 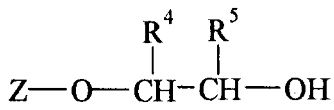

dans laquelle les définitions de $R^4$, $R^5$ correspondent à celles données ci-dessus dans la revendication 8; avec un réactif(**Wop**) de formule définie dans la revendication 8 supra.
et, de préférence puis,
avec un réactif (**Woe**) de formule également définie dans la revendication 8 supra;
pour obtenir un produit (**I.2**) :

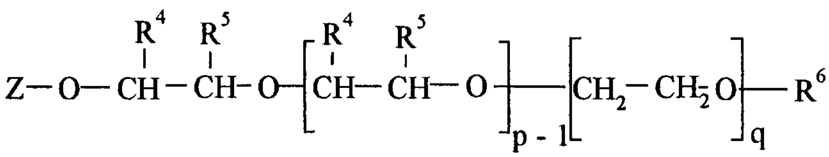

**(I.2)**

avec p, q et $R^6$ tels que définis dans la revendication 1.

et en ce que l'on effectue cette réaction en présence d'une quantité efficace de catalyseur et à une température supérieure à 100° C, de préférence comprise entre 120° et 250° C et, plus préférentiellement encore, comprise entre 150° C et 200°C.

**10.** Procédé selon la revendication 8 ou 9, caractérisé en ce que la réaction (**I'**) ou (**I"**) + (**Wop** et **Woe**) est éventuellement suivie d'une fonctionnalisation visant à transformer l'hydrogène terminal en l'un des autres substituants $R^6$, tels que définis dans la revendication 1.

**11.** Procédé selon l'une quelconque des revendications 8 à 10, caractérisé en ce que le catalyseur est choisi parmi les catalyseurs homogènes ou hétérogènes du type basique ou acide, les hydroxydes et/ou les alcoxydes de métaux alcalino-tetreux étant préférés.

**12.** Procédé d'obtention des produits (**I'**) selon la revendication 8, caractérisé en ce qu'il consiste, essentiellement, en une condensation entre au moins un bicyclo[a.b.c]-n-alkylène heptane (avec n = 1 à 6 et a + b + c = 5 ) et au moins un composé carbonylé, ladite condensation, s'effectuant, avantageusement, en l'absence de solvant et, éventuellement, en présence de catalyseur.

**13.** Composition tensioactive, caractérisée en ce qu'elle comprend au moins l'un des composés selon l'une quelconque des revendications 1 à 7,

**14.** Composition tensioactive selon la revendicaiton 13, caractérisée en ce qu elle est non-moussante.

**15.** Composition tensioactive selon la revendication 13 ou 14 caractérisée en ce qu'elle possède un pouvoir tensioactif ajustable par l'intermédiaire du nombre p + q des motifs récurrents correspondants des formules (**I**), (**I.1**), (**I.2**).

**16.** Composition parfumante, caractérisée en ce qu'elle comprend au moins l'un des composés selon l'une quelconque des revendications 1 à 7.

**17.** Composition parfumante selon la revendication 15, caractérisée en ce qu'elle possède une note parfumante modulable selon le nombre p + q des motifs récurrents correspondants des formules (**I.1**) et (**I.2**).

**18.** Composition tensioactive et parfumante, caractérisée en ce qu'elle comprend au moins l'un des composés selon

l'une quelconque des revendications 1 à 7.

**19.** Formulation détergente caractérisée en ce qu'elle contient de la composition tensioactive selon la revendication 13, 14 ou 15 et/ou de la composition parfumante selon la revendication 16 ou la revendication 17 et/ou de la composition tensioactive et parfumante selon la revendication 18.

## Claims

**1.** Compounds of the following formula:

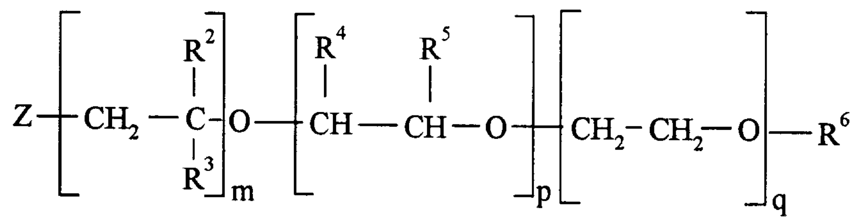

**(I)**

in which:

* Z is a bicyclo[a,b,c]heptenyl or bicyclo[a,b,c]heptyl radical, where:

- ⊕ a + b + c = 5,
- ⊕ a = 2, 3 or 4,
- ⊕ b = 2 or 1 and
- ⊕ c = 0 or 1,

said radical optionally being substituted by at least one $C_1$-$C_6$-alkyl, preferably a methyl, and comprising a skeleton selected from those mentioned below (**$Z_1$** to **$Z_7$**) and from their heptyl analogues without the double bond:

1) 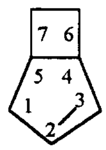 [3.2.0]

2) 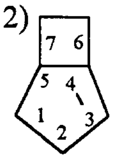 [3.2.0]

3) 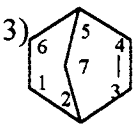 [2.2.1]

4) 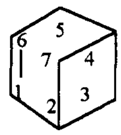 [3.1.1]

5) 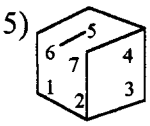 [3.1.1]

6) 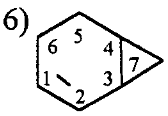 [4.1.0]

7) 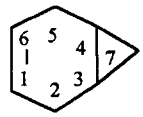 [4.1.0]

- $R^2$ and $R^3$ are identical or different and are hydrogen or a linear or branched (cyclo)alkyl or (cyclo)alkenyl group, preferably a linear or branched $C_1$-$C_{22}$-(cyclo)alkyl group, hydrogen and methyl being more particularly preferred;
- $R^4$ and $R^5$ are identical or different and are hydrogen or a linear or branched (cyclo)alkyl or (cyclo)alkenyl radical, preferably a linear or branched $C_1$-$C_{22}$-(cyclo)alkyl radical, hydrogen, methyl and ethyl being more particularly preferred;
- $R^6$ is hydrogen or one of the following groups: $SO_3M$, $OPO_3(M)_2$, $-(CH_2)_m-COOM$, where m is between I and 6, or $-(CH_2)_z-SO_3M$, where z = 1 to 6, in which groups M = H, Na, K, Li or $N(R^7)_4^+$, where $R^7$ = H or a $C_1$-$C_{22}$-(cyclo)alkyl, preferably a $C_1$-$C_6$-(cyclo)alkyl, which is optionally hydroxylated, hydrogen, methyl and hydroxyethyl being preferred; and
-
  - m = 0 or 1,
  - $1 \leq p \leq 20$, preferably $1 \leq p \leq 10$ and particularly preferably $1 \leq p \leq 3$, and
  - $1 \leq q \leq 200$, preferably $1 \leq q \leq 50$ and particularly preferably $1 \leq q \leq 10$,
- with the proviso that when m = 0, at least one of the substituents $R^4$ and $R^5$ is other than hydrogen.

**2.** Compounds according to Claim 1, characterized in that their radical Z is substituted on at least one of its carbons by at least two $C_1$-$C_6$-alkyl groups, preferably by two methyls on $C_7$.

**3.** Compounds according to Claim 1 or 2, characterized in that m = 1 (compounds **Ia**) and in that their radical Z is selected from the list comprising the radicals $Z_3$ to $Z_7$ as defined in Claim 1.

**4.** Compounds according to Claim 3, characterized in that Z = $Z_4$ or $Z_5$.

**5.** Compounds according to Claim 1 or 2, characterized in that m = 0 (compounds **Ib**) and in that the radical Z comprises the heptyl analogue, without the double bond, of the skeleton $Z_3$.

**6.** Compounds according to Claim 5, characterized in that the skeleton of formula (**I**) is substituted by at least one alkyl radical - advantageously a $C_1$-$C_6$-alkyl radical - and preferably by at least one methyl, on carbon 2 or 5 of the bicycle.

7. Compounds according to any one of Claims 1 to 6, characterized in that the repeat units of formula **(I)**, identified by the parameters p and q, are present in such an amount and arranged in such a way as to form a random or block polymer chain, preferably a block polymer chain with the order given in formula **(I)**.

8. Process for the preparation of the compounds according to any one of Claims 1 to 4 and 7 in which m = 1 in formula **(I)**, characterized in that:

- it consists essentially in reacting a reactant of formula **(I')**:

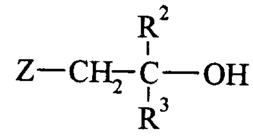

**(I')**

with a reactant **(Wop)** of the formula

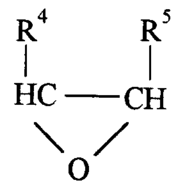

and preferably then with a reactant **(Woe)** of the formula

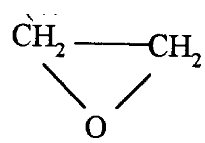

in which formulae:

- ▷ Z is as defined in Claims 1, 2, 3 or 4,
- ▷ $R^2$ and $R^3$ are identical or different and are hydrogen or a linear or branched $C_1$-$C_{22}$-(cyclo)alkyl or -(cyclo)alkenyl, hydrogen and methyl being more particularly preferred, and
- ▷ $R^4$ and $R^5$ are identical or different and are hydrogen or a linear or branched (cyclo)alkyl or (cyclo)alkenyl radical, preferably a linear or branched $C_1$-$C_{22}$-(cyclo)alkyl radical, hydrogen, methyl and ethyl being more particularly preferred, with the proviso that at least one of the substituents $R^4$ and $R^5$ is other than hydrogen,

to give a product **(I.1)**:

**(I.1)**

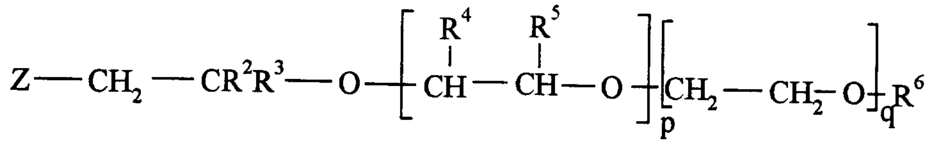

where p, q and $R^6$ are as defined in Claim 1,

- and this reaction is carried out in the presence of an effective amount of a catalyst and at a temperature above 100°C, preferably of between 120° and 250°C and particularly preferably of between 150°C and 200°C

**9.** Process for the preparation of the compounds according to any one of Claims 1, 2, 5, 6 or 7 in which m = 0 in formula (**I**), characterized in that:

- it consists essentially in reacting a reactant of the formula

(**I''**) 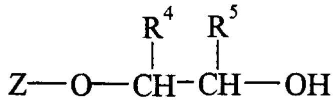

in which the definitions of $R^4$ and $R^5$ correspond to those given above in Claim 8, with a reactant (**Wop**) of the formula defined in Claim 8 above, and preferably then with a reactant (**Woe**), also of the formula defined in Claim 8 above, to give a product (**L2**):

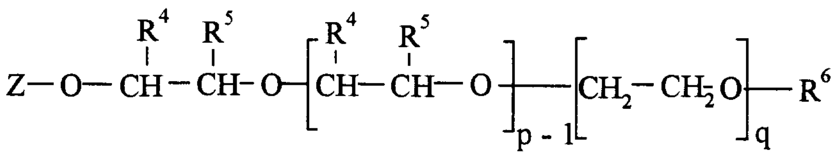
(**I.2**)

where p, q and $R^6$ are as defined in Claim 1,
- and this reaction is carried out in the presence of an effective amount of a catalyst and at a temperature above 100°C, preferably of between 120° and 250°C and particularly preferably of between 150°C and 200°C.

**10.** Process according to Claim 8 or 9, characterized in that the reaction (**I'**) or (**I''**) + (**Wop** and **Woe**) is optionally followed by a functionalization reaction, the purpose of which is to convert the terminal hydrogen to one of the other substituents $R^6$ as defined in Claim 1.

**11.** Process according to any one of Claims 8 to 10, characterized in that the catalyst is selected from homogeneous or heterogeneous catalysts of the basic or acidic type, alkaline earth metal hydroxides and/or alkoxides being preferred.

**12.** Process for the preparation of the products (**I'**) according to Claim 8, characterized in that it consists essentially of a condensation reaction between at least one bicyclo[a.b.c]-n-alkyleneheptane (where n = 1 to 6 and a + b + c = 5 ) and at least one carbonyl compound, said condensation reaction advantageously being carried out in the absence of a solvent and optionally in the presence of a catalyst.

**13.** Surfactant composition, characterized in that it comprises at least one of the compounds according to any one of Claims 1 to 7.

**14.** Surfactant composition according to Claim 13, characterized in that it is non-foaming.

**15.** Surfactant composition according to Claim 13 or 14, characterized in that its surfactant power is adjustable via the number p + q of corresponding repeat units in formulae (**I**), (**I.1**) and (**I.2**).

**16.** Fragrant composition, characterized in that it comprises at least one of the compounds according to any one of Claims 1 to 7.

**17.** Fragrant composition according to Claim 15, characterized in that its fragrant note can be modified according to the number p + q of corresponding repeat units in formulae (**I.1**) and (**I.2**).

**18.** Surfactant and fragrant composition, characterized in that it comprises at least one of the compounds according to any one of Claims 1 to 7.

**19.** Detergent formulation, characterized in that it contains the surfactant composition according to Claim 13, 14 or 15

and/or the fragrant composition according to Claim 16 or Claim 17 and/or the surfactant and fragrant composition according to Claim 18.

**Patentansprüche**

1. Verbindungen der folgenden Formel:

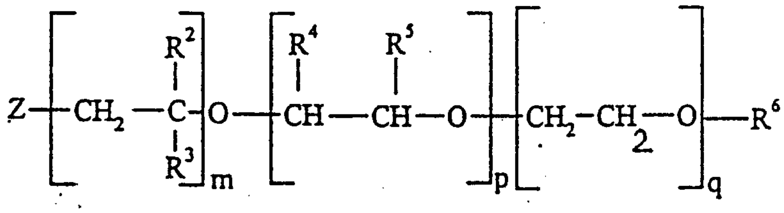

(I),

worin

* Z eine Bicyclo[a,b,c]heptenylgruppe oder Bicyclo [a,b,c]heptylgruppe bedeutet, mit:

⊕ a + b + c = 5,
⊕ a = 2, 3 oder 4,
⊕ b = 2 oder 1,
⊕ c = 0, 1,

wobei diese Gruppe gegebenenfalls mit mindestens einer $C_{1-6}$-Alkylgruppe, vorzugsweise einer Methylgruppe, substituiert ist und ein Gerüst aufweist, das ausgewählt ist unter den im folgenden angegebenen Gerüsten ($Z_1$ bis $Z_7$) sowie unter den entsprechenden Heptylgerüsten ohne Doppelbindung:

1) 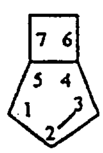
[3.2.0]

2) 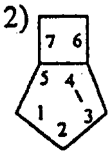
[3.2.0]

3) 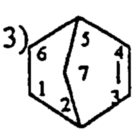
[2.2.1]

4) 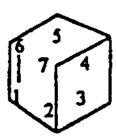
[3.1.1]

5) 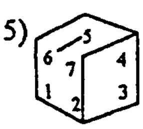
[3.1.1]

6) 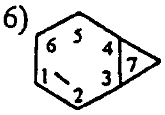 [4.1.0]

7) 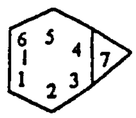 [4.1.0]

;

> $R^2$ und $R^3$ identisch oder voneinander verschieden sind und Wasserstoff oder eine geradkettige oder verzweigte (Cyclo)alkylgruppe oder (Cyclo)alkenylgruppe, vorzugsweise eine geradkettige oder verzweigte $c_{1-22}$-(Cyclo)alkylgruppe, bedeuten, wobei Wasserstoff und Methyl besonders bevorzugt werden;

> $R^4$ und $R^5$ identisch oder voneinander verschieden sind und Wasserstoff oder eine geradkettige oder verzweigte (Cyclo)alkylgruppe oder (Cyclo)alkenylgruppe, vorzugsweise eine geradkettige oder' verzweigte $C_{1-22}$-(Cyclo)alkylgruppe, bedeuten, wobei Wasserstoff, Methyl und Ethyl besonders bevorzugt werden;

> $R^6$ Wasserstoff oder einer der folgenden Gruppen entspricht: $SO_3M$, $OPO_3(M)_2$, $-(CH_2)_m$-COOM, wobei m im Bereich von 1 bis 6 liegt, $-(CH_2)_zSO_3M$ mit z = 1 bis 6, wobei M = H, Na, K, Li oder $N(R^7)_4^+$ mit $R^7$ = H oder $C_{1-22}$-(Cyclo)alkyl, vorzugsweise $C_{1-6}$-(Cyclo)alkyl, das gegebenenfalls hydroxyliert ist, wobei Wasserstoff, Methyl und Hydroxyethyl bevorzugt werden;

>

. m = 0, 1,
. $1 \leq p \leq 20$, vorzugsweise $1 \leq p \leq 10$ und noch bevorzugter $1 \leq p \leq 3$,
. $1 \leq q \leq 200$, vorzugsweise $1 \leq q \leq 50$ und noch bevorzugter $1 \leq q \leq 10$;

> mit der Maßgabe, daß mindestens einer der Substituenten $R^4$ und $R^5$ von Wasserstoff verschieden ist, wenn m Null ist.

**2.** Verbindungen nach Anspruch 1, dadurch gekennzeichnet, daß die Gruppe Z an mindestens einem der Kohlenstoffatome mit mindestens zwei $C_{1-6}$-Alkylgruppen, vorzugsweise mit zwei Methylgruppen an C-7, substituiert ist.

**3.** Verbindungen nach Anspruch 1 oder 2, dadurch gekennzeichnet, daß m = 1 (Verbindungen Ia) und dadurch, daß die Gruppe Z ausgewählt ist unter den in Anspruch 1 definierten Gruppen $Z_3$ bis $Z_7$.

**4.** Verbindungen nach Anspruch 3, dadurch gekennzeichnet, daß Z = $Z_4$ oder $Z_5$.

**5.** Verbindungen nach Anspruch 1 oder 2, dadurch gekennzeichnet, daß m = 0 (Verbindungen Ib) und dadurch, daß die Gruppe Z das dem Gerüst $Z_3$ entsprechende Heptylgerüst ohne Doppelbindung aufweist.

**6.** Verbindungen nach Anspruch 5, dadurch gekennzeichnet, daß das Gerüst der Formel (I) an dem Kohlenstoffatom 2 oder 5 des Bicyclus mit mindestens einer Alkylgruppe - vorteilhaft einer $C_{1-6}$-Alkylgruppe - , vorzugsweise mit mindestens einer Methylgruppe, substituiert ist.

**7.** Verbindungen nach einem der Ansprüche 1 bis 6, dadurch gekennzeichnet, daß die wiederkehrenden Einheiten der Formel (I), die durch die Parameter p und q angegeben werden, in einer solchen Menge vorliegen und so zusammengefügt sind, daß sie eine statistische oder sequenzielle Polymerkette bilden, vorzugsweise eine Polymerkette, deren Sequenz die in der Formel (I) angegebene Reihenfolge aufweist.

**8.** Verfahren zur Herstellung der Verbindungen nach einem der Ansprüche 1 bis 4 und 7, für die in der Formel (I) m 1 ist,
dadurch gekennzeichnet,

- daß es im wesentlichen darin besteht, ein Reagens der Formel (I'):

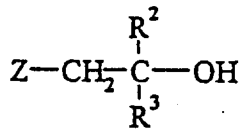

(I')

mit einem Reagens (Wop) der Formel:

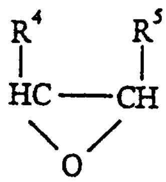

(Wop)

und vorzugsweise dann mit einem Reagens (Woe) der Formel:

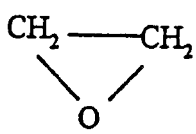

(Woe)

umzusetzen, wobei in den Formeln

> Z die in den Ansprüchen 1, 2, 3 oder 4 angegebenen Bedeutungen aufweist;

> $R^2$ und $R^3$ identisch oder voneinander verschieden sind und Wasserstoff, eine geradkettige oder verzweigte $C_{1-22}$- (Cyclo) alkylgruppe oder $C_{1-22}$- (Cyclo) alkenylgruppe bedeuten, wobei Wasserstoff und Methyl besonders bevorzugt werden;

> $R^4$ und $R^5$ identisch oder voneinander verschieden sind und Wasserstoff oder eine geradkettige oder verzweigte (Cyclo)alkylgruppe oder (Cyclo)alkenylgruppe, vorzugsweise eine geradkettige oder verzweigte $C_{1-22}$- (Cyclo) alkylgruppe, bedeuten, wobei Wasserstoff, Methyl und Ethyl besonders bevorzugt werden, mit der Maßgabe, daß mindestens einer der Substituenten $R^4$ und $R^5$ von Wasserstoff verschieden ist;

um ein Produkt (I.1) herzustellen:

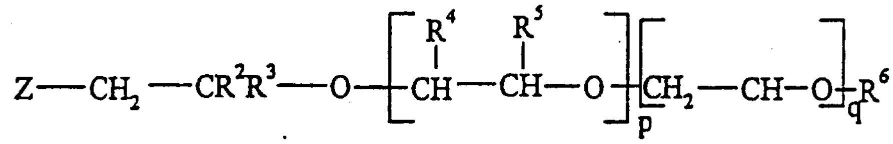

(I.1),

worin p, q und $R^6$ die in Anspruch 1 angegebenen Bedeutungen aufweisen,

- und dadurch, daß diese Umsetzung in Gegenwart einer wirksamen Menge eines Katalysators und bei einer Temperatur über 100 °C, vorzugsweise im Bereich von 120 °C bis 250 °C und noch bevorzugter im Bereich von 150 °C bis 200 °C durchgeführt wird.

**9.** Verfahren zur Herstellung der Verbindungen nach einem der Ansprüche 1, 2, 5, 6 oder 7, für die in der Formel (I) m Null ist,
dadurch gekennzeichnet,

- daß es im wesentlichen darin besteht, ein Reagens der Formel

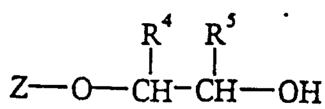

(I''),

worin die Definitionen von $R^4$ und $R^5$ den oben in Anspruch 8 angegebenen Definitionen entsprechen,
mit einem Reagens (Wop) der oben in Anspruch 8 definierten Formel
und vorzugsweise dann
mit einem Reagens (Woe) der ebenfalls oben in Anspruch 8 definierten Formel
umzusetzen,
um ein Produkt (I.2) herzustellen:

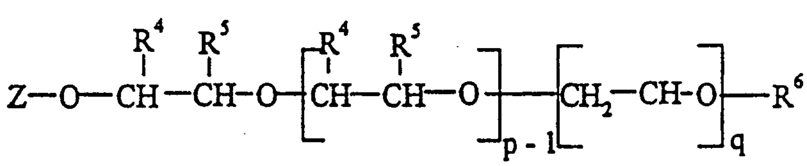

(I.2),

worin p, q und $R^6$ die in Anspruch 1 angegebenen Bedeutungen aufweisen,

- und dadurch, daß diese Umsetzung in Gegenwart einer wirksamen Menge eines Katalysators und bei einer Temperatur über 100 °C, vorzugsweise im Bereich von 120 °C bis 250 °C und noch bevorzugter im Bereich von 150 °C bis 200 °C durchgeführt wird.

**10.** Verfahren nach Anspruch 8 oder 9, dadurch gekennzeichnet, daß nach der Umsetzung (I') oder (I'') + (Wop und Woe) gegebenenfalls eine Funktionalisierung durchgeführt wird, um den endständigen Wasserstoff in einen der anderen in Anspruch 1 definierten Substituenten $R^6$ zu überführen.

11. Verfahren nach einem der Ansprüche 8 bis 10, dadurch gekennzeichnet, daß der Katalysator unter den homogenen oder heterogenen Katalysatoren vom basischen oder sauren Typ ausgewählt ist, wobei Hydroxide' und/oder Alkoxide von Erdalkalimetallen bevorzugt werden.

12. Verfahren zur Herstellung der Produkte (I') nach Anspruch 8, dadurch gekennzeichnet, daß es im wesentlichen in einer Kondensation mindestens eines Bicyclo[a,b,c]-n-alkylen-heptan (mit n = 1 bis 6 und a + b + c = 5) mit mindestens einer Carbonylverbindung besteht, wobei die Kondensation vorteilhaft in Abwesenheit eines Lösungsmittels und gegebenenfalls in Gegenwart eines Katalysators erfolgt.

13. Grenzflächenaktive Zusammensetzung, dadurch gekennzeichnet, daß sie mindestens eine der Verbindungen nach einem der Ansprüche 1 bis 7 enthält.

14. Grenzflächenaktive Zusammensetzung nach Anspruch 13, dadurch gekennzeichnet, daß sie nicht schäumend ist.

15. Grenzflächenaktive Zusammensetzung nach Anspruch 13 oder 14, dadurch gekennzeichnet, daß sie grenzflächenaktive Eigenschaften aufweist, die durch die Anzahl p + q der entsprechenden wiederkehrenden Einheiten der Formeln (I), (I.1) und (I.2) einstellbar sind.

16. Parfümierende Zusammensetzung, dadurch gekennzeichnet, daß sie mindestens eine der Verbindungen nach einem der Ansprüche 1 bis 7 enthält.

17. Parfümierende Zusammensetzung nach Anspruch 15, dadurch gekennzeichnet, daß sie eine Parfümnote aufweist, die gemäß der Anzahl p + q der entsprechenden wiederkehrenden Einheiten der Formeln (I.1) und (I.2) veränderbar ist.

18. Grenzflächenaktive und parfümierende Zusammensetzung, dadurch gekennzeichnet, daß sie mindestens eine der Verbindungen nach einem der Ansprüche 1 bis 7 enthält.

19. Reinigende Formulierung, dadurch gekennzeichnet, daß sie eine grenzflächenaktive Zusammensetzung nach Anspruch 13, 14 oder 15 und/oder eine parfümierende Zusammensetzung nach Anspruch 16 oder Anspruch 17 und/oder die grenzflächenaktive und parfümierende Zusammensetzung nach Anspruch 18 enthält.

FIG.1

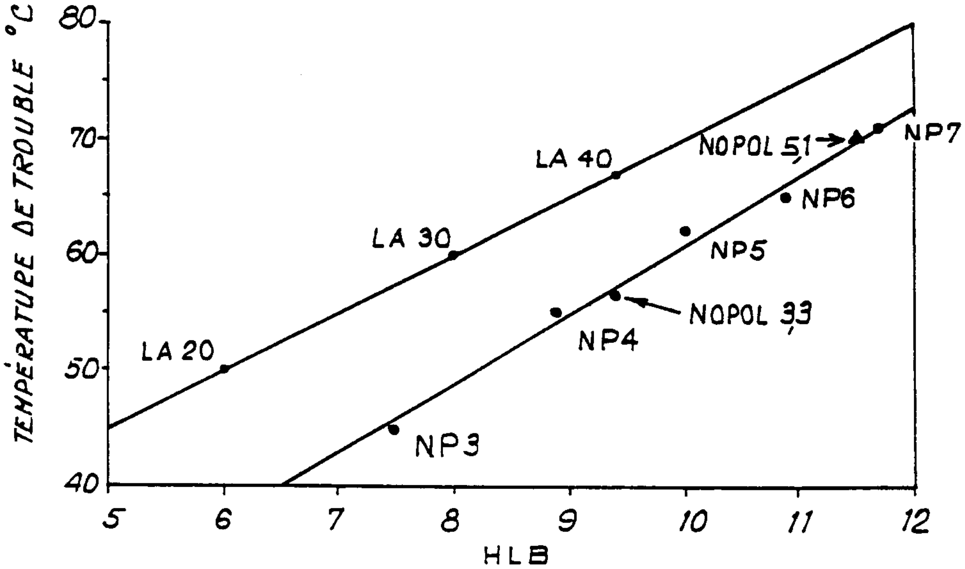


FIG. 2

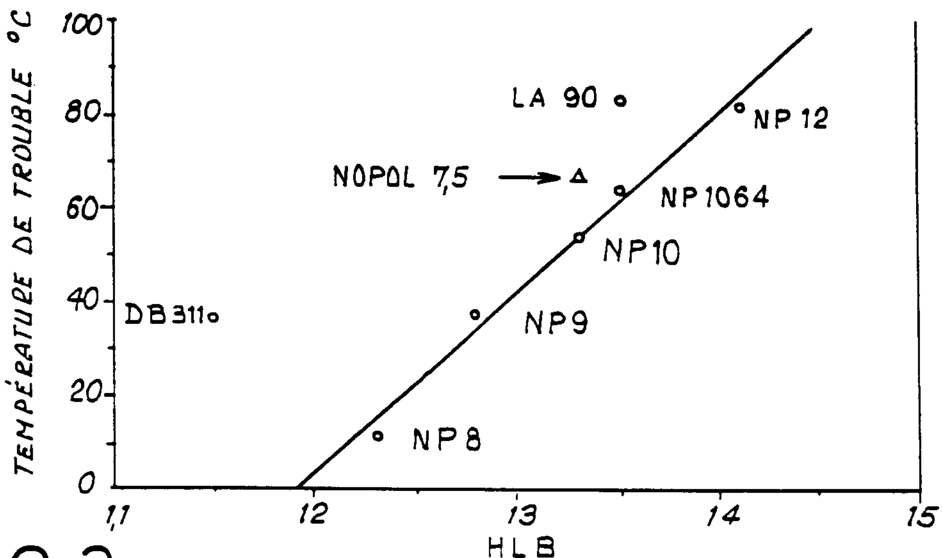


FIG.3

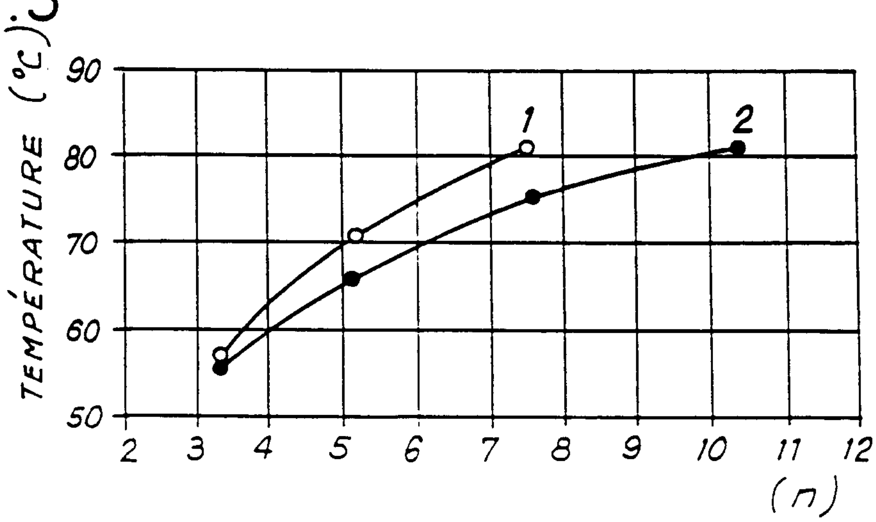

FIG.4

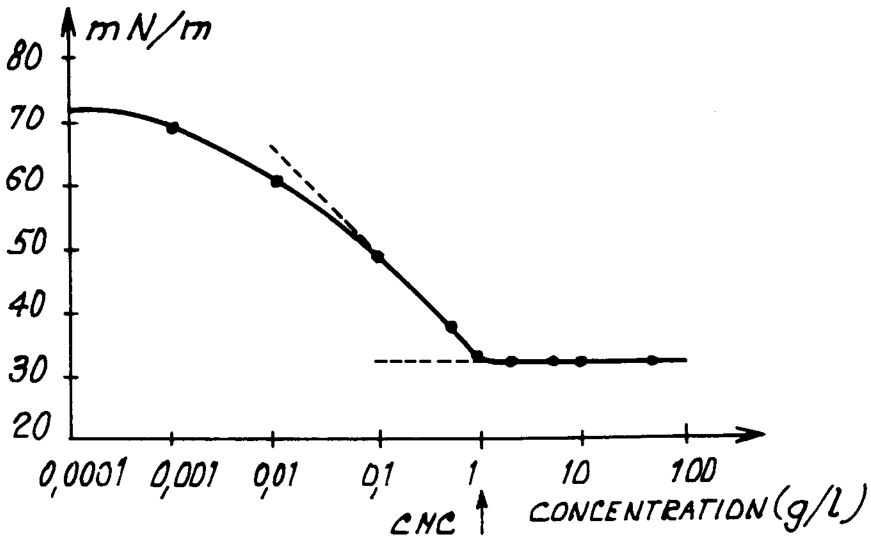
mN/m
80
70
60
50
40
30
20
0,0001
0,001
0,01
0,1
1
10
100
CMC
CONCENTRATION (g/l)

FIG.5

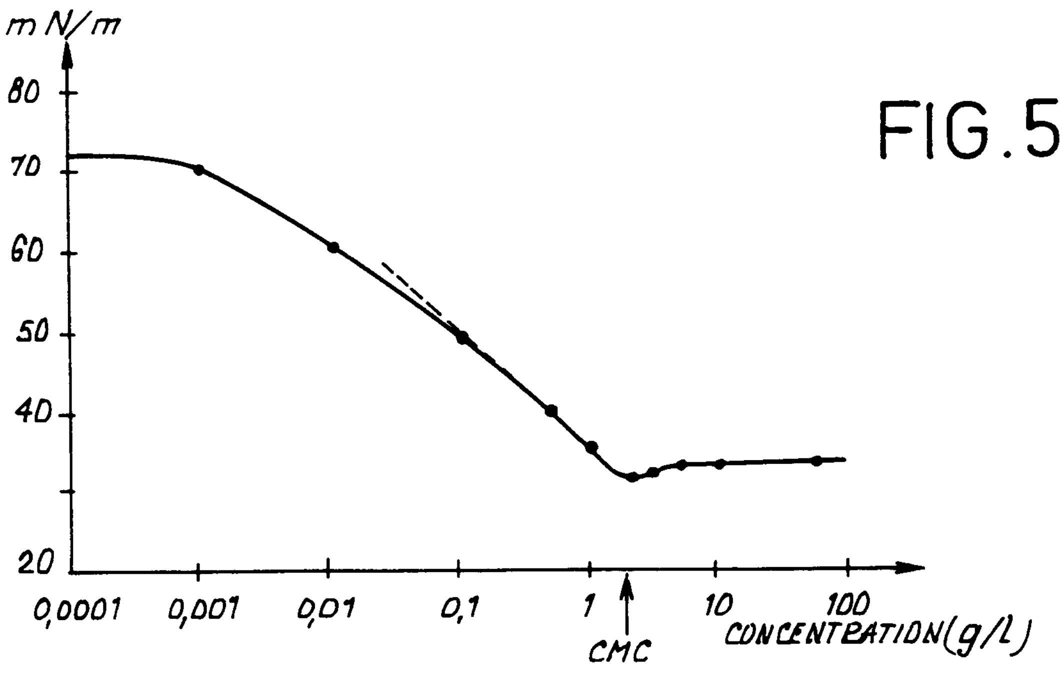
mN/m
80
70
60
50
40
20
0,0001
0,001
0,01
0,1
1
10
100
CMC
CONCENTRATION(g/l)

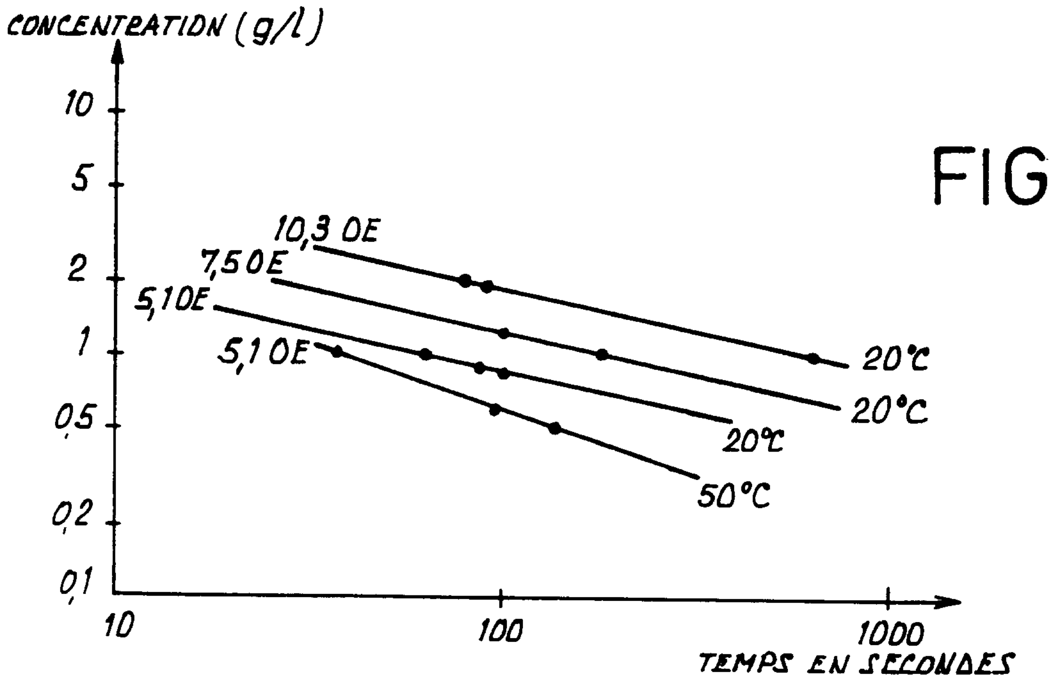
CONCENTRATION (g/l)
10
5
2
1
0,5
0,2
0,1
10
100
1000
TEMPS EN SECONDES
10,3 OE
7,5 OE
5,1 OE
5,1 OE
20°C
20°C
20°C
50°C

FIG.6

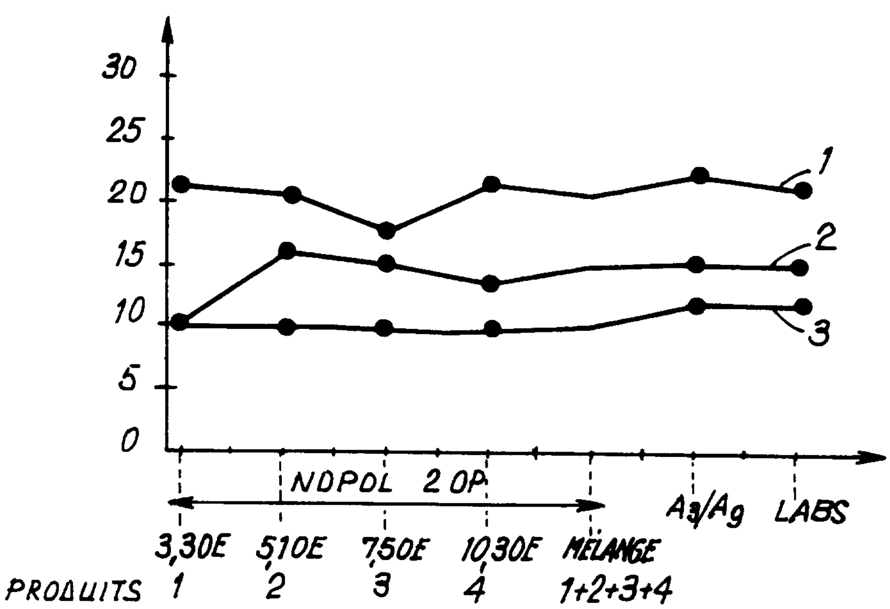
30
25
20
15
10
5
0
1
2
3
NOPOL 2 OP
A3/Ag
LABS
3,3 OE
5,1 OE
7,5 OE
10,3 OE
MELANGE
PRODUITS
1
2
3
4
1+2+3+4

FIG.7